(19) Europäisches Patentamt
European Patent Office
Office européen des brevets

(11) **EP 4 435 118 A2**

(12) **EUROPEAN PATENT APPLICATION**

(43) Date of publication:
**25.09.2024 Bulletin 2024/39**

(51) International Patent Classification (IPC):
***C12Q 1/6869*** *(2018.01)*

(21) Application number: **24177800.0**

(22) Date of filing: **06.11.2020**

(52) Cooperative Patent Classification (CPC):
(C-Sets available)
**C12Q 1/6806; C12N 15/1003;** C12Q 1/6804

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2019 US 201962931411 P**

(62) Document number(s) of the earlier application(s) in accordance with Art. 76 EPC:
**20884272.4 / 4 055 183**

(71) Applicants:
• **University Health Network**
**Toronto, ON M5G 2C4 (CA)**
• **Van Andel Research Institute**
**Grand Rapids, MI 49503 (US)**

(72) Inventors:
• **WILSON, Samantha L**
**Toronto (CA)**
• **SHEN, Shu Yi**
**Markham (CA)**

• **DINIZ DE CARVALHO, Daniel**
**Toronto (CA)**
• **HOFFMAN, Michael M.**
**Toronto (CA)**
• **TRICHE, Timothy J.**
**Grand Rapids (US)**

(74) Representative: **Avidity IP**
**Broers Building**
**Hauser Forum**
**21 JJ Thomson Avenue**
**Cambridge CB3 0FA (GB)**

Remarks:
•The complete document including Reference Table(s) and the Sequence Listing(s) can be downloaded from the EPO website
•This application was filed on 23-05-2023 as a divisional application to the application mentioned under INID code 62.
•Claims filed after the date of filing of the application / after the date of receipt of the divisional application (Rule 68(4) EPC).

(54) **SYNTHETIC SPIKE-IN CONTROLS FOR CELL-FREE MEDIP SEQUENCING AND METHODS OF USING SAME**

(57)     There is described herein, a method of capturing and analyzing cell-free methylated DNA in a sample. The method involves subjecting the sample to library preparation to permit subsequent sequencing of the cell-free methylated DNA. A predetermined amount of control synthetic DNA fragments are added to the sample. The control synthetic DNA fragments each have a known nucleic acid sequence that does not align to a target genome sequence, and at least some of the control synthetic DNA fragments are methylated. The sample is denatured, and cell-free methylated DNA and the control synthetic DNA fragments are captured using a binder selective for methylated polynucleotides. The captured DNA is amplified and sequenced.

EP 4 435 118 A2

(52) Cooperative Patent Classification (CPC): (Cont.)

C-Sets
**C12Q 1/6806, C12Q 2522/101, C12Q 2535/122,**
**C12Q 2537/164, C12Q 2545/101;**
C12Q 1/6804, C12Q 2535/122, C12Q 2537/164,
C12Q 2545/101

**Description**

**FIELD OF THE INVENTION**

The invention relates to the field of methylated DNA immunoprecipitation-sequencing and, more specifically, to methods of absolute quantification of cell-free methylated DNA.

**BACKGROUND OF THE INVENTION**

Methylated DNA immunoprecipitation-sequencing (MeDIP-seq) is becoming popular to measure DNA methylation. While cell-free methylated DNA immunoprecipitation-sequencing (cfMeDIP-seq) is robust for measuring DNA methylation at hypermethylated regions, there can be biological and technical variation that may influence results. Additionally, MeDIP-seq experiments traditionally quantifies read counts relative to experiment. This can contribute to lack of reproducibility and makes it difficult to compare results between different studies.

**SUMMARY OF INVENTION**

According to one aspect a method of capturing and analyzing cell-free methylated DNA in a sample is provided, the method comprising the steps of: a) subjecting the sample to library preparation to permit subsequent sequencing of the cell-free methylated DNA; b) adding a predetermined amount of a set of control synthetic DNA fragments, wherein the control synthetic DNA fragments each have a known nucleic acid sequence that does not substantially align to a target genome sequence, and wherein at least some of the control synthetic DNA fragments in the set are methylated; c) denaturing the sample; d) capturing cell-free methylated DNA and the control synthetic DNA fragments using a binder selective for methylated polynucleotides; and e) amplifying and sequencing the captured cell-free methylated DNA and the control synthetic DNA fragments.

In accordance with another aspect, a method of identifying a sequence for a control synthetic DNA fragment is provided for use in capturing and analysis of cell-free methylated DNA, the method comprising the steps of: a) generating nucleic acid sequences based on a plurality of target fragment lengths, a target combined guanine and cytosine (G+C) content, and a target number of CpG dinucleotides for each fragment; and b) eliminating generated sequences that align to a human genome; wherein the plurality of target fragment lengths comprises 3 to 7 target fragment lengths that are between 50 to 500 base pairs (bp); wherein the target G+C content is between 0% to 100%; and wherein the target number of CpG dinucleotides for each fragment is between 0 and ½ of the length of the fragment in base pairs.

**BRIEF DESCRIPTION OF FIGURES**

Embodiments of the invention may best be understood by referring to the following description and accompanying drawings. In the drawings:

**Figure 1** shows an experimental design for pilot testing of a set of synthetic spike-in control fragments.

**Figure 2** shows an experimental design for determining an amount of spike-in synthetic controls by spiking into HCT116 cell line.

**Figure 3** shows data transformation of fragment length. (A.) Fragment length before transformation. (B.) Fragment length after z-score normalization.

**Figure 4** shows data transformation of number of CpGs within a fragment. (A.) CpG distribution before cube root transformation. (B.) CpG distribution after cube root transformation.

**Figure 5** shows DNA methylation specificity of the cfMeDIP-seq method.

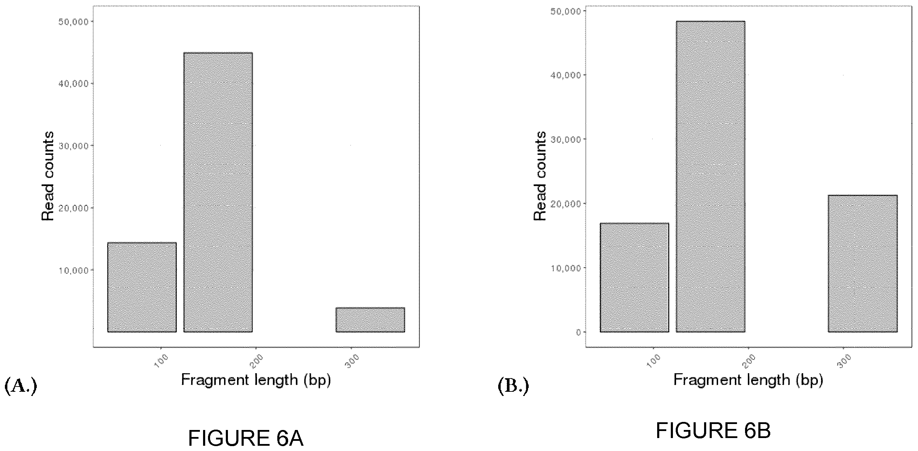

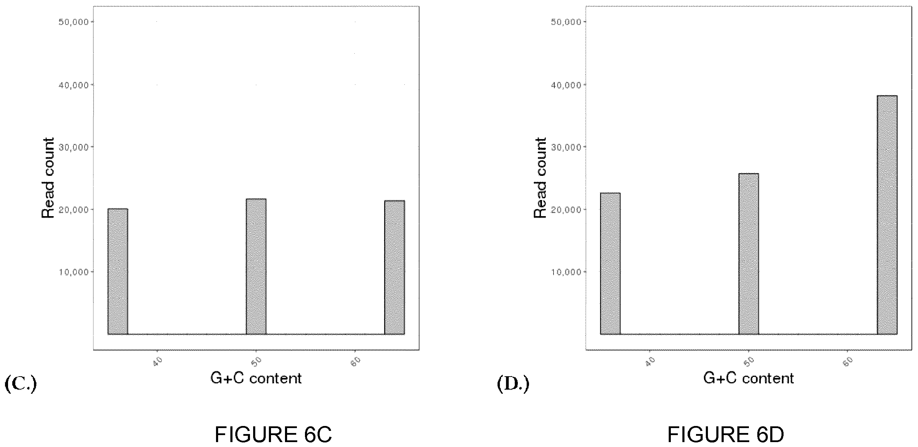

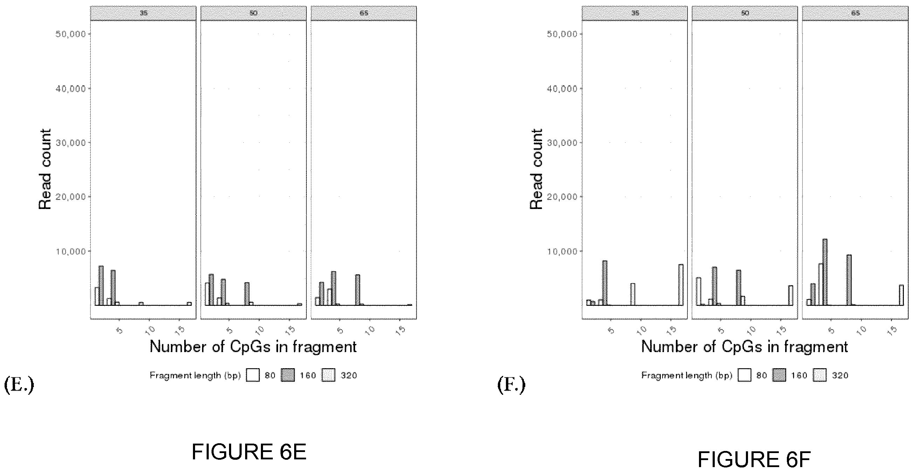

**Figure 6** shows results from sequencing synthetic DNA fragments only. Graphs show distributions of read counts with fragment length, G+C content, and number of CpGs within fragment. (A.) Fragment length distribution of unique methylated input samples. (B.) Fragment length distribution of unique methylated output samples. (C.) G+C content distribution of unique methylated input samples. (D.) G+C content distribution of unique methylated output samples. (E.) Number of CpGs in fragment distribution of unique methylated input samples, faceted by G+C content. (F.) Number of CpGs in fragment distribution of unique methylated output samples, faceted by G+C content.

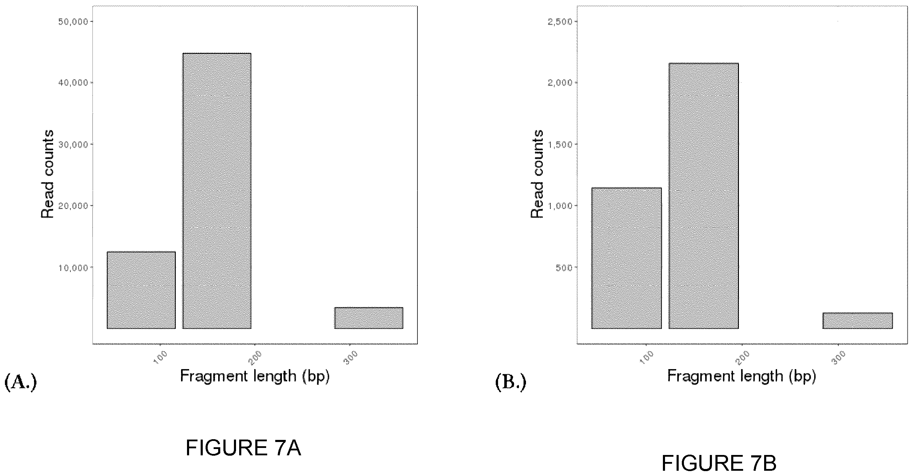

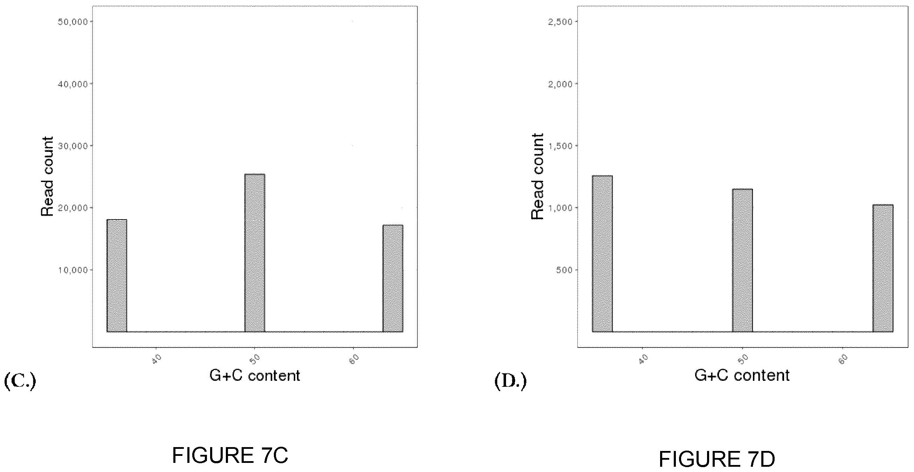

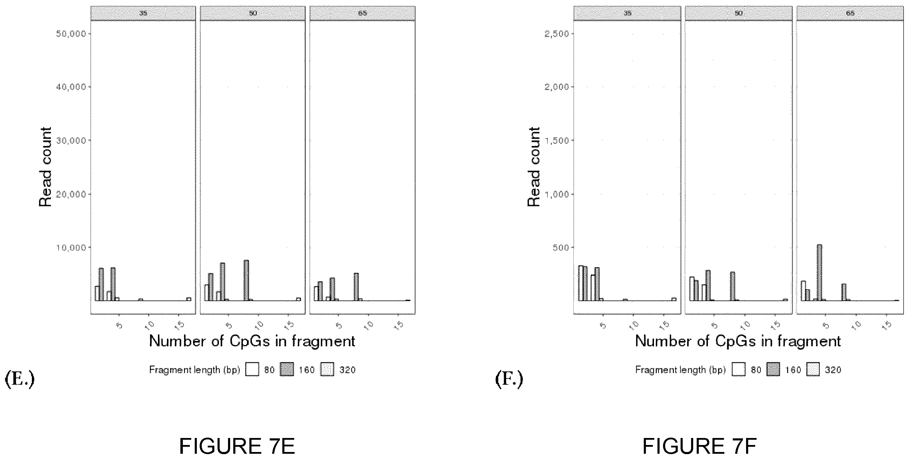

**Figure 7** shows results from sequencing synthetic DNA fragments only. Graphs show distributions of read counts with fragment length, G+C content, and number of CpGs within fragment. (A.) Fragment length distribution of unique unmethylated input samples. (B.) Fragment length distribution of unique unmethylated output samples. (C.) G+C content distribution of unique unmethylated input samples. (D.) G+C content distribution of unique unmethylated output samples. (E.) Number of CpGs in fragment distribution of unique unmethylated input samples, faceted by G+C content. (F.) Number of CpGs in fragment distribution of unique unmethylated output samples, faceted by G+C content.

**Figure 8** shows a comparison of number of reads used for spike-in controls (black bars) compared to number of reads used for biological samples, HCT116 (white bars).

**Figure 9** shows DNA methylation specificity of cfMeDIP-seq with spike-in to HCT116 on the MiSeq, 1 million reads.

**Figure 10** shows DNA methylation specificity of cfMeDIP-seq with spike-in to HCT116 on the NovaSeq, 60 million reads per sample.

**Figure 11** shows a comparison of total reads used for synthetic spike in controls (black bars) compared to biological sample, HCT116 (white bars).

**Figure 12** shows Bland-Altman plot depicting performance of a Gaussian generalized mixed model compared to known molality. X-axis: mean values between the calculated and known molality values. Y-axis: variance between the calculated and known concentration values. Bold dotted lines: 95% confidence intervals. Light dotted lines: 95% confidence interval margins.

**Figure 13** shows experimental design using synthetic spike-in control DNA to (A) assess technical bias and (B) optimize the synthetic DNA amount.

**Figure 14** shows assessing biases in fragment length, G+C content, and CpG fraction in input, output and 0.01 ng spike-in of synthetic DNA.

**Figure 15** shows Correlation between (A) picomoles and standard deviation and (B) picomoles and mappability score.

**Figure 16** shows correlation between calculated picomoles and M-values and between read counts and M-values.

**Figure 17** shows association between known variables and principal components. Left) Proportion of variance explained by each principal component. Right) Association between known technical and clinical variables to each principal component. *** p < 0.001.

## DETAILED DESCRIPTION

A cell-free methylated DNA immunoprecipitation-sequencing (cfMeDIP-seq) method was developed to work with low input DNA and with circulating cell-free DNA (cfDNA). The cfMeDIP-seq method measures DNA methylation using low input cfDNA, making it ideal for liquid biopsy applications. The DNA methylation profiles obtained from cfMeDIP-seq helps to provide tissue of origin information, important in circulating tumour DNA studies.[1-6] Similar to classical enrichment protocols that are immunoprecipitation based and sequencing protocols such as RNA-seq, interpretation requires a reference or control for comparison. Reference controls have consisted of spike-in reference DNA fragments of known sequence.[7-11]

Spike-in controls overcome the assumption that DNA or RNA yields are equal in different experimental conditions and across all genomic regions.[8] As a result, spike-in controls also adjust for biological and technical bias. The addition of spike-in controls drastically changes the interpretation of RNA-seq, ChIP-seq and genomic sequencing results.[7-11] It has been suggested that all genome-wide analyses would benefit from the addition of spike-in controls.[8] Normalizing data by total number of reads per sample often masks differences in the variable of interest. Normalizing data to assume reference control DNA is the same between samples, allows for more accurate detection of differences and adjustment of biological variables that can influence results.[8,9] While DNA and RNA sequencing experiments have utilized spike-in controls, enrichment methods of measuring genome-wide DNA methylation have not.

Here the present inventors have introduced new synthetic spike-in DNA controls to be used for cfMeDIP-seq.

In some embodiments, the spike-in controls correct for fragment length, G+C content and CpG fraction, and can be used to assess non-specific binding, an integral part of cfMeDIP-seq analysis. In some embodiments, spike-in controls with unique molecular index (UMI) were designed to adjust for polymerase chain reaction (PCR) bias, fragment length, combined guanine and cytosine (G+C) content, and number of CpG dinucleotides (CpG) per fragment. These modifications generate a quantitative measure of methylated DNA, rather than relative read counts and help mitigate batch effects.

The spike-in controls are used in sequencing methods such as cfMeDIP-seq (cell-free methylated DNA immunoprecipitation and high-throughput sequencing). CfMeDIP-seq is used to perform genome-wide DNA methylation mapping using cell-free DNA.

As used herein, "methylated DNA" refers to DNA having methyl groups added as well as derivatives thereof. For example, oxidized derivatives of methylated cytosine (5-methylcytosine) are derived through the 5mC oxidation pathway, and include 5-hydroxymethylcytosine, 5-formylcytosine and 5-carboxylcytosine (see Song, et al. Trends Biochem Sci. 2013 Oct; 38(10): 480-484, the entire content of which is incorporated hereby in reference).

According to one aspect, a method of capturing and analyzing cell-free methylated DNA in a sample is provided. The method comprises:

a. subjecting the sample to library preparation to permit subsequent sequencing of the cell-free methylated DNA;

b. adding a predetermined amount of a set of control synthetic DNA fragments, wherein the control synthetic DNA fragments each have a known nucleic acid sequence that does not substantially align to a target genome sequence, and wherein at least some of the control synthetic DNA fragments in the set are methylated;

c. denaturing the sample;

d. capturing cell-free methylated DNA and the control synthetic DNA fragments using a binder selective for methylated polynucleotides; and

e. amplifying and sequencing the captured cell-free methylated DNA and the control synthetic DNA fragments.

In some embodiments, this method further comprises the step of calculating an amount, a concentration, or a molality of the cell-free methylated DNA in the sample based on the sequenced control synthetic DNA fragments.

Cell-free methylated DNA is DNA that is circulating freely in the bloodstream and are methylated at various known regions of the DNA. Samples, for example, plasma samples, can be taken to analyze cell-free methylated DNA.

As used herein, "library preparation" includes end-repair, A-tailing, adapter ligation, or any other preparation performed on the cell-free DNA to permit subsequent sequencing of DNA.

As used herein, a "target genome sequence" refers to a genome to which the cell-free methylated DNA in the sample will be sequenced against. In some embodiments, the target genome is a human genome. In other embodiments, the target genome is a non-human genome. As used herein, a "nucleic acid sequence that does not substantially align to a target genome sequence" refers to sequences having less than 30%, less than 20%, less than 10%, less than 5%, less than 3%, or less than 1% identity in an alignment to a target genome sequence. A nucleic acid sequence that does not substantially align to a target genome sequence has no more than 2, no more than 3, no more than 4, no more than 5, no more than 6, no more than 7, no more than 8, no more than 9, or no more than 10 aligned nucleotides identical to a target genome sequence.

Various sequencing techniques are known to the person skilled in the art, such as polymerase chain reaction (PCR) followed by Sanger sequencing. Also available are next-generation sequencing (NGS) techniques, also known as high-throughput sequencing, which includes various sequencing technologies including: Illumina (Solexa) sequencing, Roche 454 sequencing, Ion torrent: Proton / PGM sequencing, SOLiD sequencing. NGS allows for the sequencing of DNA and RNA much more quickly and cheaply than the previously used Sanger sequencing. In some embodiments, said sequencing is optimized for short read sequencing.

DNA samples may be denatured, for example, using sufficient heat.

In some embodiments, the set of control synthetic DNA fragments comprises a plurality of fragments having different predetermined lengths. In some embodiments, the set of control synthetic DNA fragments comprises between 3 to 7 predetermined fragment lengths, between 3 to 6 predetermined fragment lengths, or between 3 to 5 predetermined fragment lengths. In one embodiment, the set of control synthetic DNA fragments comprises 3 predetermined fragment lengths.

In some embodiments, the control synthetic DNA fragments are 50 to 500 base pairs (bp) in length, preferably 80 to 320 bp in length. In some embodiments, a set of synthetic DNA fragments has fragments of increasing lengths. In one embodiment, a set has three predetermined lengths of 100 bp, 150bp, and 300 bp. In other embodiments, a set of synthetic DNA fragments has fragments that are multiples of a shortest fragment length. In one embodiment, a set has three predetermined lengths of 80 bp, 160 bp, and 320 bp.

As used herein, combined guanine and cytosine content (G+C content) refers to the percentage of nucleotides in a DNA fragment that are guanine or cytosine. In some embodiments, the control synthetic DNA fragments have a G+C content of between 0% to 100%, preferably between 25% to 75%. In one embodiment, where a set has three predetermined fragment lengths, the three predetermined fragment lengths have a G+C content of 35%, 50%, and 65%, respectively.

As used herein, a CpG dinucleotide (or CpG site) is a region of DNA where a cytosine nucleotide is followed by a guanine nucleotide in the linear sequence of bases along its 5' → 3' direction. In some embodiments, each of the control synthetic DNA fragments comprises a number of CpG dinucleotides ranging between 0 and ½ of the length of the fragment in base pairs. In some embodiments, each of the control synthetic DNA fragments comprises 1 to 25 CpG dinucleotides, preferably 1 to 16 CpG dinucleotides. In some embodiments, the control synthetic DNA fragments have 1, 2, or 4 CpG sites per shortest fragment length. In some embodiments, the control synthetic DNA fragments have one CpG site per 20 bp, per 40 bp or per 80 bp.

In some embodiments, the control synthetic DNA fragments have a nucleic acid sequence as set forth in on or more of SEQ ID NO: 1-59.

In some embodiments, the method further comprises:

> i. sequencing the captured cell-free methylated DNA and the control synthetic DNA fragments;

> ii. comparing the sequenced cell-free methylated DNA against the known nucleic acid sequences of the control synthetic DNA fragments; and

> iii. comparing any unmatched DNA from (ii) against the target genome sequence.

In some embodiments, some of the control synthetic DNA fragments in the set are methylated, while some of the control synthetic DNA fragments in the set are not methylated. In one embodiment, half of the control synthetic DNA fragments in the set are methylated, and the other half are unmethylated. In one embodiment, all of the control synthetic DNA fragments are methylated.

In one embodiment, the set of control synthetic DNA fragments comprise a first sequence that is methylated, and a second sequence that is unmethylated.

In one embodiment, the method further comprises estimating the amount of captured cell-free methylated DNA before amplification using unique molecular identifier (UMI) adapters.

In some embodiments, the binder is a protein comprising a methyl-CpG-binding domain. One such exemplary protein is MBD2 protein. As used herein, "methyl-CpG-binding domain (MBD)" refers to certain domains of proteins and enzymes that is approximately 70 residues long and binds to DNA that contains one or more symmetrically methylated CpGs. The MBD of MeCP2, MBD1, MBD2, MBD4 and BAZ2 mediates binding to DNA, and in cases of MeCP2, MBD1 and MBD2, preferentially to methylated CpG. Human proteins MECP2, MBD1, MBD2, MBD3, and MBD4 comprise a family of nuclear proteins related by the presence in each of a methyl-CpG-binding domain (MBD). Each of these proteins, with the exception of MBD3, is capable of binding specifically to methylated DNA.

In other embodiments, the binder is an antibody and capturing cell-free methylated DNA comprises immunoprecipitating the cell-free methylated DNA using the antibody. As used herein, "immunoprecipitation" refers a technique of precipitating an antigen (such as polypeptides and nucleotides) out of solution using an antibody that specifically binds to that particular antigen. This process can be used to isolate and concentrate a particular protein or DNA from a sample and requires that the antibody be coupled to a solid substrate at some point in the procedure. The solid substrate includes for examples beads, such as magnetic beads. Other types of beads and solid substrates are known in the art.

One exemplary antibody is 5-methylcytosine antibody. For the immunoprecipitation procedure, in some embodiments at least 0.05 $\mu$g of the antibody is added to the sample; while in more preferred embodiments at least 0.16 $\mu$g of the antibody is added to the sample. To confirm the immunoprecipitation reaction, in some embodiments the method described herein further comprises the step of adding a second amount of control DNA to the sample after step (b).

Other exemplary antibodies are 5-hydroxymethylcytosine antibody, 5-formylcytosine antibody, and 5-carboxylcytosine antibody.

In some embodiments, the sample has less than 100 ng of cell-free DNA, and the method further comprises adding a first amount of filler DNA to the sample, wherein at least a portion of the filler DNA is methylated. The filler DNA consisted of amplicons similar in size to an adapter-ligated cfDNA library and is composed of unmethylated and *in vitro* methylated DNA at different methylation levels. The addition of this filler DNA serves a practical use, allowing for the normalization of input DNA amount to 100 ng. This ensures that the downstream protocol remains the same for all samples regardless of the amount of available cfDNA.

As used herein, "filler DNA" can be noncoding DNA or it can consist of amplicons.

In some embodiments, the first amount of filler DNA comprises about 5%, 10%, 20%, 30%, 40%, 50%, 60%, 70%, 80%, 90%, or 100% methylated filler DNA with remainder being unmethylated filler DNA. In preferred embodiments, the first amount of filler DNA comprises about 50% methylated filler DNA. In some embodiments, between 5% and 50%, between 10%-40%, or between 15%-30% are methylated filler DNA

In some embodiments, the first amount of filler DNA is from 20 ng to 100 ng. In preferred embodiments, 30 ng to 100 ng of filler DNA. In more preferred embodiments 50 ng to 100 ng of filler DNA. When the cell-free DNA from the sample and the first amount of filler DNA are combined together, there comprises at least 50 ng of total DNA, and preferably at least 100 ng of total DNA.

In some embodiments, the filler DNA is 50 bp to 800 bp long. In preferred embodiments, 100 bp to 600 bp long; and in more preferred embodiments 200 bp to 600 bp long.

The filler DNA is double stranded. The filler DNA may also be endogenous or exogenous DNA. For example, the filler DNA is non-human DNA, and in preferred embodiments, λ DNA. As used herein, "λ DNA" refers to *Enterobacteria* phage λ DNA. In some embodiments, the filler DNA has no alignment to human DNA.

In other embodiments, methods of identifying a sequence for a control synthetic DNA fragment is provided. The control synthetic DNA fragment is then used in capturing and analysis of cell-free methylated DNA. The method involves:

a. generating nucleic acid sequences based on a plurality of target fragment lengths, a target combined guanine and cytosine (G+C) content, and a target number of CpG dinucleotides for each fragment; and

b. eliminating generated sequences that align to a human genome;

The plurality of target fragment lengths comprises 3 to 7 different target fragment lengths that are multiples of a unit length that is also the shortest fragment. A fragment length is between 50 to 500 base pairs (bp), preferably 80 to 320 bp. The target G+C content is between 0% to 100%, preferably between 25% to 75%. The target number of CpG sites for each fragment is between 0 and ½ of the length of the fragment in base pairs, and 1, 2, or 4 CpG sites per shortest fragment length. In some embodiments, the target number of CpG dinucleotides is 1-25, preferably 1-16 CpG dinucleotides per fragment. In some embodiments, the control synthetic DNA fragments have one CpG site per 20 bp, per 40 bp or per 80 bp.

In one embodiment, the method generates nucleic acid sequences that has three target fragment lengths that are 80 bp, 160 bp, or 320 bp, and the target G+C content is 35%, 50%, or 65%, respectively.

The following examples are illustrative of various aspects of the invention, and do not limit the broad aspects of the invention as disclosed herein.

## EXAMPLES

### Methods

To meet the need for a reference control in cfMeDIP-seq experiments, spike-in controls were designed with integrated use of unique molecular indexes (UMIs) to adjust for polymerase chain reaction (PCR) bias, and immunoprecipitation bias caused by the fragment length, G+C content, and CpG density of the DNA fragments. This enables for absolute quantification of methylated DNA in picomoles, while retaining epigenomic information that allows for sensitive, tissue-specific detection as well as comparable results between different experiments. 54 DNA fragments were designed with combinations of methylation status (methylated and unmethylated), fragment length in base pair (bp) (80 bp, 160 bp, 320 bp), G+C content (35%, 50%, 65%), and fraction of CpGs within a fragment (1/80 bp, 1/40 bp, 1/20 bp). Spike-in control DNA sequence were checked to ensure they had no cross alignment to the human genome and minimized formation of secondary structures to avoid issues with amplification. The cfMeDIP-seq was carried out on either solely spike-in DNA fragments, spike-in DNA added to sheared HCT116 genomic DNA or spike-in DNA added to cfDNA from the stored plasma of acute myeloid leukemia (AML) patient samples to assess technical and biological biases, determine optimal amount of spike-in DNA required for an experiment and to assess batch effects, respectively.

*Designing synthetic DNA spike-in controls.* Spike-in controls were designed with unique molecular index (UMI) to adjust for polymerase chain reaction (PCR) bias, fragment length, G+C content, and number of CpGs per fragment to allow for absolute quantification rather than relative read counts.

Paired-end sequencing data previously generated by the cfMeDIP-seq protocol[6] was used to assess the different properties of cfDNA to aid in the design of synthetic controls[12]. The number of CpGs was assessed as an integer of fragment length in base pairs (i.e. 1 CpG in 80 bp fragment is comparable to 2 CpGs in a 160 bp fragment). Using the distribution of cfDNA properties including: fragment length or size, G+C content and CpG fraction, the following spike-in fragment parameters were set as shown in Table 1. Number of CpGs within a fragment were set as an integer of fragment length [1/80, 1/40, 1/20].

**Table 1.** Parameters for synthetic spike-in control fragments.

| Fragment length | 80 bp | 160 bp | 320 bp |
|---|---|---|---|
| G+C content | 35% | 50% | 65% |
| CpGs | 1/80 | 1/40 | 1/20 |

First, 27 different first-order Markov model was used to generate sequences with these exact parameters[5]. GenRGenS v.2.0 was used to generate the sequences.[13] BLASTn was used to ensure no alignment to the human reference genome (GRCh38/hg38), choosing sequences with the highest E-values possible. Integrated DNA Technologies (IDT) UNAFold™ software (IDT, Coralville, USA) was used to check for secondary DNA structure for 80 bp and 160 bp fragments.[4] UNAFold does not support sequences over 280 bp, therefore, RNAstructure™ software[8] was used to check for secondary DNA structures of the 320 bp fragments. For each Markov model (N=27) numerous sequences were generated, picking two from each model that fulfilled the criteria for both lack of alignment to human genome and lack of potential secondary structures. Two distinct fragment sequences were designed for each combination of parameters: one to be methylated and one to be unmethylated to assess specificity of binding to 5-methylcytosine. 52 synthetic DNA spike-in controls [(9 (80 bp) + 8 (160 bp) + 9 (320 bp)) × 2 = 52] were used to assess biases in the cfMeDIP-seq method due to variation in fragment length, G+C content and CpG number.

*Synthetic fragment preparation.* The 80 bp and 160 bp fragments were ordered as 4 nmol Ultramer™ DNA Oligo and the 320 bp fragments as gBlocks Gene Fragments (Integrated DNA technologies, Coralville, USA). The sequences are listed in Table 2. Each fragment was PCR amplified using High-Fidelity 2X Master Mix (New England Biolabs, Ipswitch, MA, USA, Cat M0492L) at its determined optimal annealing temperature (see Table 2). Amplified fragments were purified using the QIAQuick PCR Purification Kit™ (Qiagen, Hilden, Germany, Cat 28104). Concentration was determined via Nanodrop™. For each methylate fragment, 1 μg of synthetic DNA fragment was taken for methylation with CpG methyltransferase (M.SssI) (ThermoFisher Scientific, Waltham, MA, USA, Cat EM0821).

The methylation reaction was incubated at 37 °C for 30 min, then 65 °C for 20 min. The methylated product was purified using the MinElute PCR Purification Kit™ (Qiagen, Hilden, Germany, Cat: 28004). To test that the fragments were properly methylated, the original PCR amplicon and the methylated PCR amplicon were digested with either HpyCH4IV, HpaII or AfeI restriction enzyme dependent on the fragment (Table 2). Methylation was considered verified when the PCR amplicon had a single band when run on a 2% agarose gel. Once the methylated fragments were verified, molar amount of synthetic fragments were measured using Qubit.

**Table 2.** Sequences of synthetic DNA spike-in controls.

| Name | SEQ ID No. | Sequence | Annealing Temperatu re of PCR product (°C) | Methylated? (Yes/No) If so, the restriction enzyme used to confirm methylation |
|---|---|---|---|---|
| 80b_1C _35G-2 | 1 | TGTCTAAATTAAAGTTGTGATCTTTG ACTTAGCATCGACTCACCCTATAGC CTACCAGACAAGAATTATGAAGAAC ATAT | 50 | |
| 80b_1C _50G-2 | 2 | GTACACCATCATTATCCTCATAGCT TAGTCTCCCGCAGGCCCAGGGTAC ATAAGGCTTGGAGATTCACTGTTAG CTGCTC | 60 | |
| 80b_ 2C_ 50G-2 | 3 | GCCTCCCCAACTATAGGGTCAGGA AGGATTATGGCACCCCACACGATTT TCACCCGATCTGTACCAGTAATCAT ACATGG | 60 | |
| 80b_1C _65G-2 | 4 | GCTACCAGTGGCCCCCCCCTACCG AGTCCCCCATTAACCTCACCCCCCT GACTGCTAACCTGGGATGGTGAAG CCTGGGC | 60 | |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 80b_ 2C_ 65G-2 | 5 | GGTTATGCCCCCGCCCTGCATCCT CCCTGTCTACACGGCCCAACCCTA GCAATGTGTGGCCCCCCCTGCTGT CTCCCATC | 60 | |
| 80b_ 4C_ 65G-2 | 6 | GCTGGTGCACCGCTGCCCCCACCC ACCTCGCTTGTCACAGCCTCGGTA GGTCCTGATTTGATGCTTGGGTGCT CGGCTGG | 60 | |
| 160b_ 4C _35G-2 | 7 | GTATAATCATAACAAAGGCCTAATG AAAGACGCTGATTTGAAACTAGTTC CCTCATCATCTGATAGATTTCCTCG TGTCTTTTTTCGTGAATGGCACAAT ATGGTGTGAAGACCTATTACAATCA AAAGTATAAACTAGCGACTAAGAT CTCAGAATTA | 60 | |
| 80b_ 1C_ 35G-1 | 8 | TAGGATATAGGTTGTCCCCTAGTAG GAGATAAACTTTGATTAACATCCAA TTGATCGTTAGTGTCCTTCAAAATTA TGCT | 60 | No |
| 80b_ 2C_ 35G-1 | 9 | TCTAATACTCATCTTAGCTCGCGTG CTTTGTGATTTTAGTGCTGAAATTCT TAAATGTTAACCACTGTGAAATCCA TAAG | 60 | No |
| 80b_ 2C_ 35G-2 | 10 | CTCAAATATAACAAGAGTAGCAAAC TTACAAAGATCGCTGACAAGTATGT TATCCATTTCTAAGCGCTACCAATA ACACT | 60 | Yes AfeI |
| 80b_ 4C_ 35G-1 | 11 | AAGGCATTACTTATCTAATCAATCG ACAAAACGTTAAGTCAGTGTTAGGA TAGTGTCATTTGTACTCGTAGACGA AATTG | 60 | No |
| 80b_ 4C_ 35G-2 | 12 | TTATTATTGACCGTACACTATTTAAC TAACAGATATGACGTATTACTATGA TATGTTAATGACGCTGAGCTGCTCG GAGA | 60 | Yes HpyCH4IV |
| 80b_ 1C_ 50G-1 | 13 | GAGGACCATATAGCTCGCACAGGA ACCAGCTGAAGAATTGATTGGTAGT GCTGACCAGACACCAACCTTCAAAC CTCTGC | 60 | No |
| 80b_ 2C_ 50G-1 | 14 | ACAACACCCTCCACCCAATACTTGT GAGTTGGTCGCAGCACGAGCCTAG TCTCCTTGTAAGTCAGTCAAATGCC TGTAAC | 60 | No |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 80b_ 4C_ 50G-1 | 15 | AGTCATCAGCATATTGTCAGTACCC AGTGGTCTCTAGGAAAATCGGCCG GTACGTAAATACTCCTAGTGGGCTG CGTGGT | 60 | No |
| 80b_ 4C_ 50G-2 | 16 | GCTTCTTATGATACCAAAGTTGCCC AAAAAGGCTAGCGTTCTAGTTAGGG TGCAGCCGCGAGAAGACCGGGTTC ATGAAG | 60 | Yes HpaII |
| 80b_ 1C_ 65G-1 | 17 | GCAGGCTGCAGGGTTTGGCCCCTG GTTCCGTTCCAGCAGGTGGCATAG GTGGGGAAAGCCAGGTGCCTACAG TGGGGTGG | 60 | No |
| 80b_ 2C_ 65G-1 | 18 | CACCTTGAGACCTCCAGAGGGGGA TCCACAACTTGCGCCCTCTGTGAAG TAGGCTCTGGTGCGCAGGGGGGAA GGGGGGC | 60 | No |
| 80b_ 4C_ 65G-1 | 19 | TTGGGAGGCTCTGGACTGGGGCAA ACGACACCGTGCATCAACTGTGTG GTGGTGGCCTCGTCCCCCCCCATC CTCTCCGC | 60 | No |
| 160b_ 2C _35G-1 | 20 | TTAGTCGAGATTTTAGCCTAATTGA GAGATAGTCCGATGATATGTCTTTG ATCTAACATGTCATCATGAAATATG AAGCCAACACACTCATATGTTCATG TGACAAAGATCCAGTTAAGCCAGT ATTGAGGTTTGTCCATCACTTAAGT ACTATTCATT | 60 | No |
| 160b_ 2C _35G-2 | 21 | CTTTACTACTGAATGTAAGCTCTTG CAGAGGATCTAACAGGGATAGAATT ATGAACACGTCTGTCACACATAACT TCAAATGCAATTTATTAATAAGGGT CAGAATGTGTGGTATCTTTCCAGAC TTATATCATTCCCTTTACTATAACCG ATTACACAT | 60 | Yes HpyCH4IV |
| 160b_ 4C_ 35G-1 | 22 | ATGTGTAAGAAATAAAATACTGGCT CATCATCATAAACTTGTCTATAATGT CACTATTATCACAAAGAATGCAGGT ACGACACGGTATCGGCAGCAGTGG ATAGCTCGTATCTATATGAATAGGG GAAGTGAATAATATGACAATAGTAT ACTTTGCTTA | 60 | No |

(continued)

| Name | SEQ ID No. | Sequence | Annealing Temperature of PCR product (°C) | Methylated? (Yes/No) If so, the restriction enzyme used to confirm methylation |
|---|---|---|---|---|
| 160b_2C_50G-1 | 23 | TTGTAGTACAGTCTAACCATCTTGA CCCAGTAGCTCCCCATCTGATATGC TCAGTAGCTAGGGTGGCCTGAGGG AACCGGTCAAACCCACTTATTCTGA ACCCCAGAGGTATGTTATGCGCAG GAACCTGCCTTCTATTGGTAGTGTC TTGGGTCAACAG | 60 | No |
| 160b_2C_50G-2 | 24 | GTAACATGGTTACCACTGGGACCG GACCTTTTCACCTCCACTTTCAGGG AATAGGATTCAGTCCTGTATAGCAG TGTGACACCCCAAGGCCAATTCCA CCCTACATTCAATGCCTGAGTGTAT GTTGGCCATTGGGTAACTAGCCGT GTCCCAACCTCAT | 60 | Yes HpaII |
| 160b_4C_50G-1 | 25 | AGCCTTGGACGTGAGTCTCTGTTTC TGACCCAACTGAGATCTTTTTACTG TCATTCTACCCCCTAGAGACTCGCG TTTCTAGAGAGGGGATGTATGTGAG GGGTTGTGATTTAGCCCGTGATGC CCTAGGATCTTGAGACAATTGTCAG GGCCCTCCAGT | 60 | Yes HpyCH4IV |
| 160b_4C_50G-2 | 26 | GGCTCTAGGGGGTGATAAAGTCTC GGATTATGCTGTATGGAGTCCCATC AACTTCCAATGACAATATTGTACTCT AGGATAGCTAGATGACGCCCCAGG CAAAGAACCCTTTTCGTATGAGGCC AGCCTTCCAAGGTCCACTAGGCTC AGCTCCTCGATG | 60 | No |
| 160b_8C_50G-1 | 27 | GGTAAGTATGCAGCTCAACGAGGG TACCGGTAGCGACCCGCTGTTTGTT ACTAGTAAGGACTCAGTATTGCGCT CTACTTGGTTCCTCATGACAGCTAT GCAGGGATGTGTTCAGCCCGTTCT ACCGAACCCTTCTAACATGAGCGTG CCTTTTGATTAG | 55 | No |
| 160b_8C_50G-2 | 28 | GCGAGTAACTGCTTCAATGGGACTA CAATGTGCCACGGGTGCCCTACAG TCCTCAGCCCCAATTGCCCAAAACG AACCTTTCAACATCATCCCGGATTT TCACTCGAAGATTGTGACTGGGGG TTTTATGCAACAACCGAGCTATTAC ATGGTTGCGCGT | 55 | Yes HpaII |
| 160b_2C_65G-1 | 29 | TTCTCAGGCAGCCCACCCCGGCAG TCCAGATCTAGCCCCCTCCCCTTG GTACTTGGGCATGGTGAGCCTCCG AGACCCCCTCCCTCTCCCCCCTCA CCAGACCCCCCCCTATAGGTCCTG | 50 | No |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| | | CAAGGTGCCTTCCCAAACACCCCA GTTAGGCATGGCCACC | | |
| 160b_ 2C _65G-2 | 30 | GACTCCTCCCTAGGCCCCCATGGA GCCACCCCCTCAGGCCACTCCAGG CTACTAGGCCCAGGTTCCAGGCAA ATGCCCTCTCTGCCAGTGCCACTA GCAACACCTCCCCTATCAAGGTGG CCCCAGGTCCTCACGTAGCATGCA GGCCCCCCGCTCCATC | 60 | Yes HpyCH4IV |
| 160b_ 4C _65G-1 | 31 | CCCCAGAGGCAGGTGCCCTACCAA GCTCCCCCCATGACCCCTAAATCC CCCACCCTGCCCCGGCGGTTGCAG TGGTACCAACCAGTCAGGCCCCTC GCCAGTACCCTTCCATATCTTCAGC CTCCTGGCCATTCGATCAGGAGCC CCACAGCCCTAGGCC | 60 | Yes HpaII |
| 160b_ 4C _65G-2 | 32 | TAGGGCCCGAGCCAGCCTGTACCT TGCGCCCTGCCCCCCCTCTACCT GGGGACCCCACGGTCATCCTTGAC AGGGTGCCCCTCGGCCCACTCCCA TTCTCCTTTTGTCTCCAGTAAACCC CCAGAGCCCAAGGTCAGCCTGCTG CAGGGTTTGCCTCCA | 60 | No |
| 160b_ 8C _65G-1 | 33 | ACTGCTGCGCGGCGCACCTCCCAC ATGTCCTACCCATCACCTCCTCAGT GTTCACTGGCTGGGTCTGTCCTCCT ACAGGGTGCCAAGCGGGGCTCCAT TGCCACTAGAAGCCCATGGTCCAG CGTGGCTAGATCCGAGCGGGGGGG CCTCCACCAGCCGTC | 60 | No |
| 160b_ 8C _65G-2 | 34 | TGGAGGGGCTGGGCCTGCTCCCCT AGTGCGGAATCCTGCCCTCCGGTG GCTTGCTCTTTGGGTCCACGGGTA CTAGAGGGGAATTATGACCAGAGC CCTGCAGCCCCGAAGCGGGGTGC GCCACAGTCCCCACGACTCCGCCA ACCTTCATACCCTGTCC | 60 | Yes HpaII |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 320b_ 4C _35G-1 | 35 | AAATGTATAAATTTGGTGAGGACTG TAATTCTAGTTGTACTCCTATGTCTA CAAGACCATCTCCTTACTATAGTGG GATTAATAATATTGTAAATCCGGCT ATGATCTTAGACAGGGAAAATGAGT TGTAACCGATTGTTAAGTATCATTTT TCCTTGAATTGACATCACCTAGCTT GTCTTAATGTTCATGAGAATTTCAG GCTAACCACAATGTCAACTATGCGA CACCATGTATCATCATTTCCACTTC ACAACAGAACCGGGTCATTTTGTGT ATTCCCATAGATTAAATGATTAACCT TATGCCACTATAATATA | 55 | No |
| 320b_ 4C _35G-2 | 36 | TAATGTATAAATATGGTGAGGACTG TAATTCTAGTTGTACTCCTATGTCTA  CAAGACCATCTCCTTACTATAGTGG GATTAATAATATTGTATATCCGGCTA TGATCTTAGACAGGGAAAATGAGTT GTAACCGATTGTTAAGTATCATAATT CCTTGAATTGACATCACCTAGCTTG TCTTAATGTTCATGAGAATTTCAGG CTAACCACAATGTCAACTATGCGAC ACCATGTATCATCATTTCCACTTCA CAACAGAACCGGGTCATAATGTGTA TTCCCATAGATTAAATGATTAACCTT ATGCCACTATAATATA | 60 | Yes  Hpall |
| 320b_ 8C _35G-1 | 37 | AGCATAAAAAGCCTATAACTCGATT TTTTAACATTAAGCGGTACCGTTTC TGCATCCAATGACATAATATATATG GGAGCTTACTATTGAGATGCACTCT TAATACGGAATTACGTTACAAGGTA GAGGGCTATGACTAGAATTGAGCTT TATATTAGCAGAAGTGTCTTGTCCA GTAGGGTCTTGAAAAGTTATTATGT ATGGTGTTCATGAGAATCGAGGTAC ATTAAGGTGAATCATTTAAATCCTA GTATGGATGTTACACTTCAATGCTT TTGTACAACTAACTCGGGTGCGTAA ATATATTGAAACAATGTTTA | 60 | Yes HpyCH4IV |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 320b_8C _35G-2 | 38 | ACTGGATTGTAGCTATGCCTAGCAT TCCTTCTCTTGAGCCTCAAAGTCTT ATCTGATGTTCATTCCAACACTCTT GAACGGATTTTAAAAACAAATATGT ATAACCGACGCAAGAATTTAATATA TGAATAAGTCTCCTGTTCTAGATTTA ATCTCAATAGTGATTATCGAAAATTA GTATAGATTTAGTGAGAATAGAGAT GTGTTCCTTCCTTAATAGTCTTTAAT CTTGGACATGGTGAGCAGTATTATA TCCGACTGTTAAGTCGAGACTGTCT TTCGTAATGTGACGCCTTACTTTTT GAGATAGAGAACCTAAG | 60 | No |
| 320b_16 C_35G-1 | 39 | TACTATAATTAGGCAGGTGATTGAA GAACCTGTTCTTTAACTATCATATAC CAGTACATACTTCAACTATTTTTGTT GATCAGGATCTATTAACGAATCACG TTTGACTTAATTTACAACTTGCTCGC GGTATTAATAAATAGATTTTATTTGC CGTGTTTTCAGTACGTATAATGCGA TCAAGCAGCAATGCCGGAACTGTTA ATTGTCCTCGCCTTACTGATATGTT TAAACTTCAACTTTTCGTCTCGAGG TTTAGTAAAATAATGTATTAAAGAAT ACGAACGTGATTATCCCGACGGCA CACTGTCACTTCGTCT | 60 | No |
| 320b_16 C_35G-2 | 40 | GAACAACTTATCTGAGAACAAGACT ACGTCAACTTTTGTACGTGGGATAA GTTTTCCTGAATTCTAATTATAAATA TGGACGTGCTCAATGAATTAACAGA CGCCACACGACGTTTATATCGGACT GATTACAAGTTTATTCTGTGTAAGTA ACAACAACGCTACGATCTCTGTATG ATTGAATACAGTCAAACGGTTCTAT AATCACTACTCATTCTACTGTTCGA ACTAATATCAATCAAGTCGTTAATAA ATCTTCATGATCACTCGCTATTTCTA GAAGTGACTCGCTAAGGACGATAAT TATTCTTCGTTCAAAA | 55 | Yes HpyCH4IV |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 320b_ 4C _50G-1 | 41 | GCTAACACCATGGCTGCTAGAATTA AAGTATTGACTGACTCATACGTGGA ATACCCAAGCCAACCCTGTCCCCTC AACTAAAGGTGATTCTGGACCTTTC AAGGGTTGGCAGGTATTTACCCCTA TCGACAAGAAGAGTGACCTACAGG AGAAATCGATCAGAGGCAGTTCAA GATCAACTCCCTGGTCCTCCTGGC CTGGAGCTCATGATGAAGAGTCCA GTGCCTCCTGCTCACCAGCATCCC ATGTGACGCAGGTCACTAGGCCTT GTGGCCTTAAAAAGCCCAGCACATA CTGACTAGGGCAGTTCAGCTTATAC A | 60 | No |
| 320b_ 4C _50G-2 | 42 | GGCTCACCAGTGGCTCCCCATCCG ATCAAGCTACCCAACTAATGTACTC AAGGTACATAATTAAGGTAGAACCA CCGAAAGTCTACTAGTGATGTTCAC TCTGTCCCTGGGATAAGAGTAGCAT AAGGCCACTAAGCTCCACTACCTCA GCCAACGTAATGTCTCTTTCCAGCG TCTGTTCAAAATGCTCTTGGTAGTG GTTCTGCTAGGTAAGGGCAGTTCCT TTGCCAGGGTCTAGACCTAGCCCA GTGTGGTTACCCCCATGAACAACA GGGTGGAGGCAGGGTCAACCCAG CTACTGGCCATAATTTCTAGAGTCA | 60 | Yes HpyCH4IV |
| 320b_ 8C _50G-1 | 43 | GCCACTGGGGACCACCTCATTACC AGGCTTTGGCATGCTGTAATGTCTC CGATCCTTGAGATGCCCTGCCCCC AATCGCAGATGTCAGTAGGCAGCT AGCACAACTGAAACTACTCCACCCC AACCGTGATCCTGGTGCAAGGCTT CCCAAAGAAAATATTTAACTAAATAC AGAGATGCTACCTAAATCCCGCTG GGAGTTAATCAGATTCGATCCGCGA CCCCCTTTGGTGTAAGAGTGTAGCT TGCTTCTTATACCCTCTCCCCGCTC CACAATAGGAGCCTACTTCACCACA CCAATAAGGTGAACATCCTATCTC | 60 | No |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 320b_8C _50G-2 | 44 | AGGGAGATCTAGCCTGGCTAAGCA GGGGCAACTAGTGCACTTCTTTCCA CTCCAGCGCACTTCACCATTAATGG ATGTACAGATGATTGTTAGTTTGAC TCTCATCAGCAATTCACTCCCTACT TACGTTTGTGGCCCCTTACGTCTAG ATATGGGGTCCGAGTAGCCCGACT GCTTTCCTCATCAGTTTTGGCAGCC TGCAACCAAACCCCAGTAGATAAAG GCAGTGTGCTACACGTCCGGGGGT AAGAAGCCTGGGTTCCTTTAACTAA GTACTCCAACCACATTACAGGGGC ATCCCGCTCAGTAGTTGATGGTCA | 60 | Yes HpyCH4IV |
| 320b_16 C_50G-1 | 45 | AGCGGGCCTAGGTTACACCCCCGC AACATTTCTAAATGCATCTAGGGAC CTTACTGGCACAGTTCAGCCCGCC AGTTAATTATCTTATTGAGATCCTGC AGAGGATAATCTCCTTTCGGAATTA CCACAACGGATTCGCGTAAACTAGT CTGCGATTGCTTTGTAAGCCAAGG GCTACACTGTATCCAGGGGCTGGA GGGTTTAGAAGTTTCCGTTCCTCAT TTCCGAAACTAGACCTGATAGGCTG TCGTGTCCACGGATTCTACCAAGCA TTCGCTCCGTACCCACTTGCTACCG ACTGTTGCCGTTGCCTACAGGTA | 60 | No |
| 320b_16 C_50G-2 | 46 | ATACCTCAGTTTATATTGGACCTCT AGCTGCCGGTTAGAGAAACTATTAG AAAGCACGGTTCTATACGGACATTC TTGGGCTGTATGATACAAACAGAGG CCGGTACGACTACTTCCGCTCCAT GTAATTGACGAAGAGTCTTCTCCCT AGAGATCGCTTATCCTTATTGCTAA TGCTTGCCATAGGCCCCGCTTAGC ACGACTGCCGATACGTGAATGCTAT TGAGTACCGGCCCAGTCGTCCCGC ATCTCCCACACTGAACCGGGTTCTC AGCTATGTCAACTGTCCTTCTTGTC TTTGGGTGCAGGGTCACCTGCCC | 60 | Yes HpyCH4IV |

(continued)

| Name | SEQ ID No. | Sequence | Annealing Temperature of PCR product (°C) | Methylated? (Yes/No) If so, the restriction enzyme used to confirm methylation |
|---|---|---|---|---|
| 320b_4C_65G-1 | 47 | TGTATGATTTGAAGAGATTTGTATAT ACACACATTGTTTTGTAGCATAGAA AAGGAGTTTTTGTCAACCGGTAGCC CACCCTGATTCTCAACCAAGCCTGT AGATCTGTAATTGGGGTCTTAAGTC CTTGTTAAATTCTGGACAGCACTAT GATTTTTTACATTCTAAATCATTATA CCAAGGTATCTTGTCTTATCTTCAG AGTGTCCAGCCTGTCGATAGATCG GAATACAATCGTATAATTAATTGTTA AGCATGTTTCTTGTACATACAGGTC AGTTACATCAACATACTTATAAACA GTGCTGTAATATTTGTGA | 50 | Yes Hpall |
| 320b_4C_65G-2 | 48 | GCAATTGATGATAACTGTGAGTGAT TTTGTTTCTTTTGGAGACTACCTAAC GCTTATGACTTTGAGTTTCTGGTAT GATTCAAAAGTAGAATACCTGTAGC ACGAGCACCAATATCATTGTTAACT GAGGAAGTTCATGTACTTACCGAAT TATAGGAAAATTCAGTAGCTTTTCTT TGCCTACTAACTTAGGTTGTGTTCA TCGAAATCATAACATCACATAACTAT TGTCTTCCATAAGCACTCAGGACTT CAAGTAAAAAGGATGAAGCCTATTC CATTTCACATCTGAATAACTTTAGCA AAGTGTAAGAAGCTAA | 60 | No |
| 320b_8C_65G-1 | 49 | CCCATGCATCAAACTGGCTCCCTC GTCTTCCGATTGTCTAGCTCATAGG CTCTCGAAGCATCTAAGGGCTATTC CGGGTCCTGCAAGTATAAGCTTCAT TATTCCTAAGGATGTGGGGAGTGA CTCAGAGGTCCAGATCGCACTGTG GGCCAGACTGACACAGCTTCAAAG GAAGGGCCTCCAAGTCCACTGCAC TCAGAATTAAGAATTCCCTGACGCA TTATCTTGAGAAAGGCACTGGTCCA TGTCTCTTGTATCTCCGAGACCAAC TTAAAGGAGGGATGGGAGCTAACA GGCACCTCCCGACTTATCTTACCAG T | 60 | Yes Hpall |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 320b_ 8C _65G-2 | 50 | GAACTTCCAAATACACCGTCCCATC TGTTCAGTCAGGGATTGGGGTGAG AGATATATCGCATCAGGAATTACGA AACCTTATGGGCAAGCAGTGATTAG CTAAGCCTGGAAACCTGGCAATTAA CACCTCAAACTGGATCCATGCCTTT CTAACTATTTCCCACCCCTTGGCAG TCTAGGGCTGGCGAGAGGCCCTGC TAATACTTGAGGCGTAGATGGGGG GCGGCTTCTCTGCAAACTGGTGCC CGCTGGGGGCTCTAATAATTATTCTT CCTTGTTCCACACAACCAGCCCCTC GACTTTAAGCAGCTATGCTATGCA | 60 | No |
| 320b_16 C_65G-1 | 51 | TTCGGCACTTGTCTGCCCTCGTCAG AAAATGTTGGGTAAAACCCTAGGTT GTAGTTTGGGTCTGGCGAGCGGGA AAGTGCATGCTCGGCCCATGTGGG CTCCAAACTGAAGGTTATTAGATTC CTAGATGGTGAGACCGCATACAAAA AGGGCCCTGGAAAGAGGTCACTTC AACGCATCTCCTGATATTGGTCTGG TATCCACAGTAGAGCTATTGTCGCC TAACAGTGATGCCGCGCCGTCCTG TATTGGTGCGCGAGACAGCTTATAC | 60 | Yes HpyCH4IV |
| | | GTACCTGAATGGCGATAATTATCCG AGGGGCAGACTCAAGCTTAAGAAA | | |
| 320b_16 C_65G-2 | 52 | TTGGGCCGCCTTGTCCGCAACCAG CAACCGATAGCAGTCGGACTCCGA GTCAGTAGTGAAGTGCTTTAGCGTT AAGTGTTTATTGTGAATGAGCCCTC TCTCCCCCAAATCACAAGAGGTGG CGGAAAAACACGAAGCCGAAGTAC ACCGACAAGGAACGGTGCTCTCAA GAGTTGCCAGCCATTGCTAGACAG AGTAATTTCCTCCTCCAGGCGGAAT TCAACAGTCCTCAGTCCCAGAATTA TCTTGGGAAAGGATGGACACGAAT ATTTGGAACAGTGGACGCCGACCC GTTTAATTACAGGGTTCCCTGAGAT TGT | 60 | No |
| 80b_ 1C_ 35G- 2_mod | 53 | TGTCTAAATTAAAGTTGTGATCTTTG ACTTAGCAACGTCTCACCCTATAGC CTACCAGACAAGAATTATGAAGAAC ATAT | 50 | Yes HpyCH4IV |
| 80b_ 1C_ 50G- 2_mod | 54 | GTACACCATCATTATCCTCATAGCT TAGGCTCCACGTGCCTACAGGGCC ATAAGGCTTGGAGATTCACTGTTAG CTGCTC | 62 | Yes HpyCH4IV |

(continued)

| Name | SEQ ID No. | Sequence | Annealing Temperatu re of PCR product (°C) | Methylated? (Yes/No) If so, the restriction enzyme used to confirm methylation |
|---|---|---|---|---|
| 80b_ 2C_ 50G-2_mod | 55 | GCCTCCCCAACTATAGGGTCAGGA AGGATTATGGCACCCCACACGTATT TCACCCGATCTGTACCAGTAATCAT ACATGG | 62 | Yes HpyCH4IV |
| 80b_ 1C_ _65G-2_mod | 56 | GCTACCAGTGGCCCCCCCCTACCG GATCCATCCCTAACCTCACCCCCCT GACTGCTAACCTGGGATGGTGAAG CCTGGGC | 62 | Yes Hpall |
| 80b_ 2C_ 65G-2_mod | 57 | GGTTATGCCCCCGCCCTGCATCCT CCCTGTCACACGTGCCCAACCCTA GCAATGTGTGGCCCCCCCTGCTGT CTCCCATC | 62 | Yes HpyCH4IV |
| 80b_ 4C_ 65G-2_mod | 58 | GCTGGTGCACCGCTGCCCCCACCC TCCACGTCTGTCACAGCCTCGGTA GGTCCTGATTTGATGCTTGGGTGCT CGGCTGG | 60 | Yes HpyCH4IV |
| 160b_ 4C _35G-2_mod | 59 | GTATAATCATAACAAAGGCCTAATG AAAGACGCTGATTTGAACATAGTTC CCTCATCATCTGATATTGTCCTACG TGTCTTTTTTCGATGAGTGCACAAT ATGGTGTGAAGACCTATTACAATCA AAAAGTATAAACTAGCGACTAAGAT CTCAGAATTA | 60 | Yes HpyCH4IV |

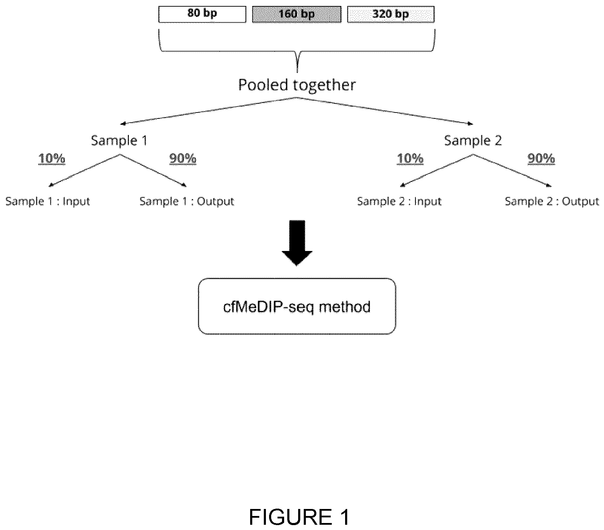

*Pilot testing.* To assess whether the spike-in controls work, cfMeDIP-seq was performed on only the spike-in controls, not spiked in to a biological sample. Within each group of fragments lengths (80 bp, 160 bp, 320 bp), the total number of synthetic fragments was added together in equimolar amounts. The samples were then pooled together in equal amounts to make up 10 ng of input DNA (3:33 ng per fragment length). CfMeDIP-seq was as per Shen et al. [7]. UMI adapters were used to account for PCR amplification bias, which required the adapter ligation to be an overnight incubation at 4 °C with final adapter concentration adjusted to 0.09 pmol. For each sample, 10% (1 ng) of the product was saved after DNA denaturation as input. Input and outputs of two replicate samples (N=4 total) were amplified followed by purification and dual size selected using AMPure XP beads for 150-200 bp. Samples were sequenced on the MiSeq Nano flowcell (Illumina, San Diego, USA), 1 million reads per flow cell at paired-end 150 bp (see Figure 1.).

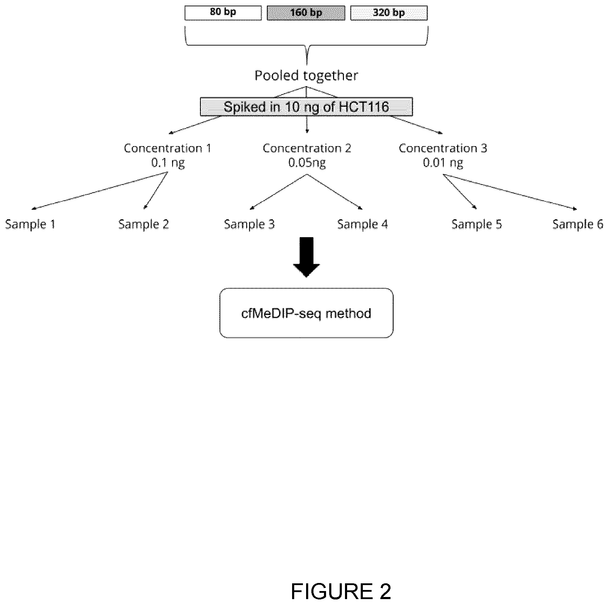

*Spike-in controls to HCT116.* In order to test the optimal concentration of the synthetic fragments to use as spike-in controls, ensuring enough DNA present in the final product to be informative but not overwhelm all the sequencing reads, it was tested by spiking in the synthetic fragments into HCT116 cell line, shearing the HCT116 genomic DNA to mimic cell-free DNA (cfDNA) that would be present in a cfMeDIP-seq experiment. The sheared HCT116 cfDNA mimic was kept at constant 10 ng, while varying the different concentrations of synthetic fragment pools, from 0.1 ng, 0.3 ng, and 1.0 ng of DNA. The samples were prepared the same way as in the pilot testing and sequenced on the MiSeq Nano, 1 million reads per flow cell, paired end 150 bp (Illumina, San Diego, USA). High resolution sequencing was then performed spiking in 0.1 ng, 0.05 ng and 0.01 ng of our control into 10 ng of HCT116 on the NovaSeq, 60 million reads per sample, paired end 2×100 bp (see Figure 2).

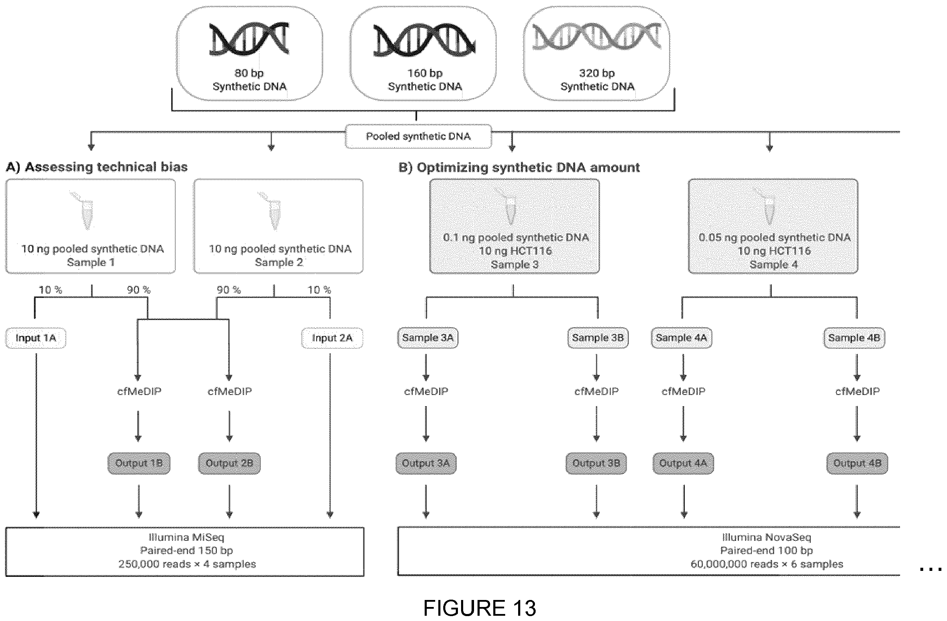

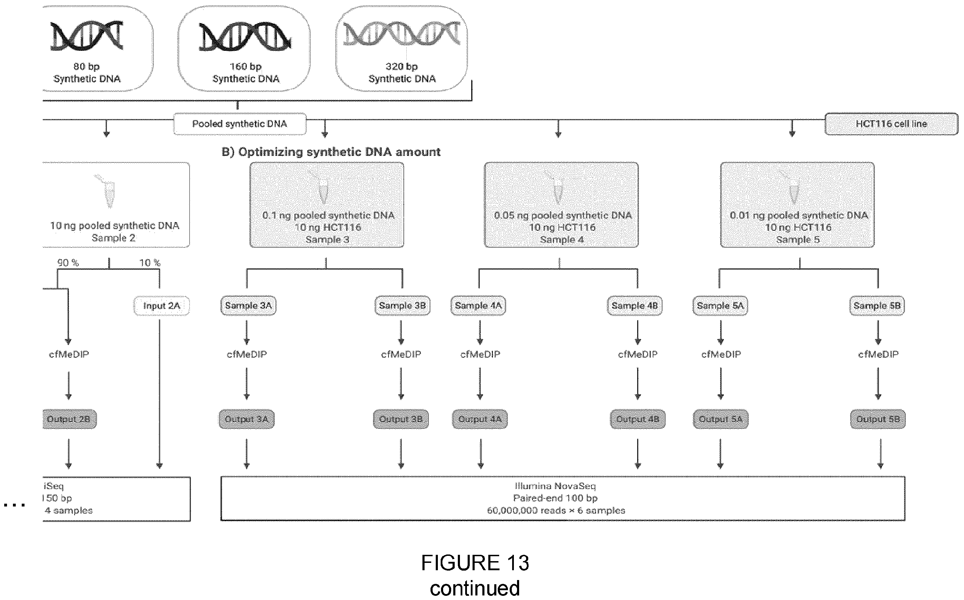

*Assessing technical bias.* To assess the performance of synthetic fragments as spike-in controls, cfMeDIP-seq was performed using the spike-in control DNA pools as the input. The input pool consisted of 9.99 ng of synthetic spike-in DNA, with equimolar amounts of each fragment size and within each fragment size pool, equimolar amounts of each methylation status (Table 2). The cfMeDIP-seq was performed as per Shen et al (2018)[2] with slight modifications. The xGen Stubby Adapter and unique dual indexing (UDI) primer pairs (Integrated DNA technologies, Coralville, IA, USA, Cat# 10005921) were used to account for PCR amplification bias. Adapter ligation was performed overnight at 4 °C with final adapter concentration adjusted to 0.09 pmol by dilution. For each sample, 1 ng of the DNA denaturation product was saved as input. For each sample, we amplified both input and outputs followed by purification and dual size selected using AMPure XP beads (Beckman Coulter, Brea, CA, USA) for 150 bp-200 bp. Samples underwent sequencing (Princess Margaret Genomics Centre, Toronto, ON, CA) on a MiSeq Nano flowcell (Illumina, San Diego, CA, USA), paired-end $2\times150$ bp, 1 million reads per flow cell (Figure 13).

*Optimizing synthetic DNA amount.* The optimal amount of spike-in control DNA needed per experiment was determined by adding varying amounts of spike-in controls to sheared HCT116 genomic DNA (ATCC, Manassas, VA, USA, RRID: CVCL_0291). The HCT116 genomic DNA, a colorectal cancer cell line, was sheared was sheared using a LE220 ultrasonicator (Covaris, Woburn, MA, USA) and size selected using AMPure XP beads (Beckman Coulter, Brea, CA, USA) to mimic cfDNA input. 3 replicate samples were created with masses of synthetic spike-in control DNA of 0.1 ng, 0.05 ng, and 0.01 ng, adding each of them to 10 ng sheared HCT116 cfDNA mimic. The cfMeDIP-seq experiment was performed as previously described in Shen et al. (2018)[1] Samples underwent sequencing (Princess Margaret Genomics Centre, Toronto, ON, CA) on an Illumina NovaSeq 6000 (Illumina, San Diego, CA, USA), paired-end $2\times100$ bp, 60 million reads per sample (Figure 13).

*Bioinformatic preprocessing.* The adapters were trimmed using fastp version 0.11.5[2] (see Formula 1) and removed reads with a phred score of less than 20. The reads were aligned to the sequences of the designed fragments using BowTie2 version 0.11.5[3] (see Formula 2). Subsequently, sequences that did not align to the present synthetic DNA were aligned to the human reference genome (GRCh38/hg38).[15] Over 98% of reads aligned to either spike-in control sequences or the human genome in every sample. Read pairs were removed when at least one read in the pair did not align or had low quality. Low quality was defined as a Phred score < 20. Reads that contained the same UMI were collapsed as counted as one read by matching the UMI sequences for each read per sample.

```
--umi --umi loc=per read --umi len=5

--adapter.sequence=AATGATACGGCGACCACCGAGATCTACACATATGCGCACACTCTTTCCCTACACGAC

--adapter sequence r2=CAAGCAGAAGACGGCATACGAGATACGATCAGGTGACTGGAGTTCAGACGTGT
```

(1)

```
bowtie2 --local -x [sequence reference] --minins 80 --maxins 320
```
(2)

*Calculating absolute concentration from spike-in control data.* Deduplicated read counts from pilot testing, along with G+C content, CpGs, fragment length and molality (pmol) were used to create a generalized linear model to calculate molality (pmol) of a given fragment within the original sample. As size selection results in a non-monotonic relationship between read count and fragment length, we transformed fragment length using Formula 3:

$$x=(160 - \text{fragment length})^2$$

(3)

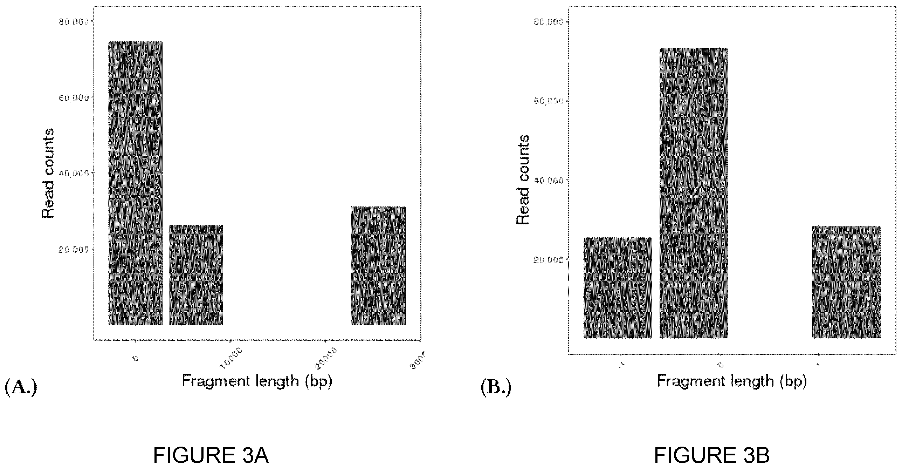

This transformation results in a left skew of the data. Hence a z-score was used to normalize these data (Figure 3). (Formula 4)

$$\left(x - \mu_{fragment\ lengths}\Big/\sigma_{fragment\ lengths}\right)$$

(4)

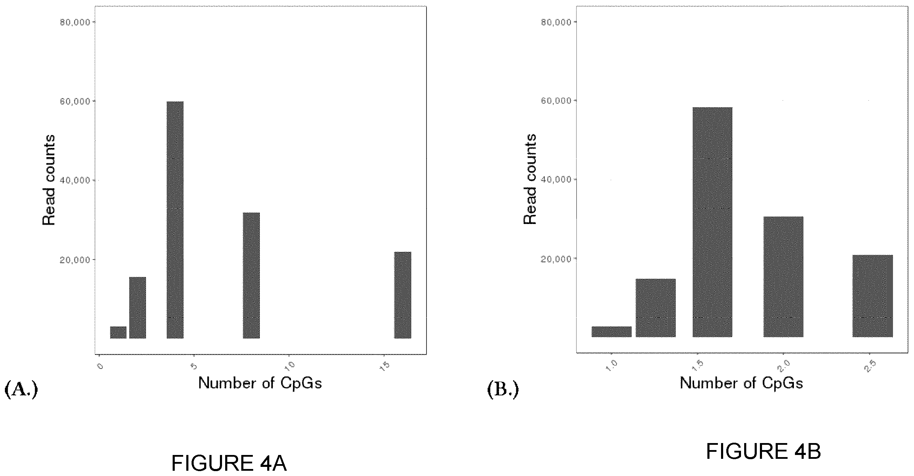

Distribution of the number of CpGs per fragment also was left skewed. To return these data to a normal distribution we used the cube root $\sqrt[3]{Number\ of\ CpGs}$ (Figure 4).

$$\text{molality(fmol/ng) per fragment} = (x - \mu_{fragment\ lengths} / \sigma_{fragment\ lengths})$$
$$+ (G + C\ content) + \sqrt[3]{Number\ of\ CpGs} + (Read\ count) \tag{5}$$

A Gaussian generalized linear model was used with log link to create a value (molality) to account for fragment length, G+C content and number of CpG which can influence the results of these data. (Formula 5) Best model to use will differ on a per experiment basis.

*Absolute quantification from spike-in control data.* A generalized linear model was created to predict molar amount from deduplicated spike-in control read counts based on UMI consensus sequence, G+C content, CpG fraction, fragment length. To do this, the stats package in R version 3.4.1. was used. To reduce its left skew, a cube root transformation of CpG fraction was used. A Gaussian generalized linear model (Equation 6) was used to calculate molar amount ($\eta$) for each DNA fragment present in the original sample using regression coefficients ($\beta$) learned for each experiment. This model includes read counts ($x_{reads}$), number of fragments ($x_{fragments}$), length of fragments ($x_{len}$), G+C content of fragments ($x_{GC}$), and CpG fraction of fragments ($\sqrt[3]{x}_{CpGfraction}$). Regression coefficients ($\beta$) for each experiment and model can be found in Table 3.

$$\eta = \beta_0 + \beta_{reads}x_{reads} + \beta_{fragments}x_{fragments} + \beta_{len}x_{len} + \beta_{GC}x_{GC} + \beta_{CpGfraction}\sqrt[3]{x}_{CpGfraction}$$
$$(6)$$

**Table 3.** Regression coefficients ($\beta$). A Gaussian generalized linear model was used for all experiments.

| | Intercept Coefficient | Fragment length coefficient | G+C content coefficient | CpG fraction coefficient | Read counts coefficient |
|---|---|---|---|---|---|
| 0.01 ng in 10 ng HCT116 | 0.0039210000 | -0.0000105700 | 0.0000030070 | 0.0001706000 | -0.0000001584 |
| Batch 1 | 0.0038410000 | -1.1420000000 | 0.0000022400 | 0.0002940000 | -0.0000001390 |
| Batch 2 | 0.0039900000 | -0.0000110500 | 0.0000012400 | 0.0001415000 | -0.0000000049 |
| Batch 3 | 0.0038670000 | -0.0000112000 | -0.0000014390 | 0.0004484000 | -0.0000001343 |

As in previous analyses,[1,5,6] the genome was binned into non-overlapping 300 bp windows. Bedtools intersect22 was used to calculate the proportion of a given fragment that overlapped with the defined 300 bp windows. An adjusted molar amount ($\eta'$) was calculated to only consider the portion of the bin each fragment overlapped. This model includes overlap between the fragment and the genomic window ($\theta$), window size (x), and the molar amount ($\eta$) from equation 7.

$$\eta' = (\theta/x) \times \eta \tag{7}$$

*Identifying regions to be filtered.* To assess the relationship between molar amount and mappability, umap k100 mappability scores were used.[23] Mappability scores were annotated to present 300 bp windows and used the minimum mappability score for every 300 bp window. Standard deviation was calculated between the two replicate samples for which 0.01 ng of synthetic DNA was spiked into 10 ng of HCT116 genomic DNA. The relationship between molar amount and mappability scores was assessed, and molar amount and standard deviation excluding simple repeat regions,[24] regions listed in ENCODE blacklist,[25] regions with mappability score $\leq 0.5$ and regions with standard deviation $\geq 0.25$. HOMER version 4.10.4 was used to investigate whether specific transcription factor binding motifs associated with our outliers. Window size was set to 300 bp and outliers were compared to the HOMER-generated randomized genomic background.

*Correlation between picomoles and M-values.* Fragment length, CpG fraction, G+C content, and read count was used to model molar amount. Molar amount ($r^2$ = 0.93) was estimated using a Gaussian generalized linear model. Models that performed better on 160 bp fragments were prioritized as a size selection was performed for these fragments, and these are the fragments of interest.

To show that quantifying methylated DNA as a molar amount in picomoles is a valid measure of DNA methylation, HCT116 genomic DNA was run in triplicate on the Illumina EPIC array (Illumina, San Diego, CA, USA). The HCT116 genomic DNA samples run on the EPIC array are technical replicates of the HCT116 genomic DNA spiked with 0.01 ng spike-in control. EPIC array data was normalized and preprocessed using sesame.[27] CpGs was annotated on the EPIC array to 300 bp genomic windows. When >1 CpG probe annotated to a window, probe M-values were averaged across the window. Windows were removed that mapped to UCSC simple repeats,[24] ENCODE blacklist,[25] regions of low mappability $\leq 0.50$, and regions where standard deviation between replicates $\geq 0.25$. Correlation was assessed between EPIC array M-values and picomoles and EPIC array M-values and read counts at windows that contained $\geq 3$ CpG probes, $\geq 5$ CpG probes, $\geq 7$ CpG probes and $\geq 10$ CpG probes.

*Examining consistency across experimental batches.* To mimic known batch effects and to test whether our spike-in controls can mitigate batch effects better than current analyses not using our spike-in controls, a sample of 10 ng of cfDNA obtained from the plasma of 5 AML patients was given with 0.01 ng of spike-in controls to three independent researchers. The AML patient samples were collected from the Leukemia Tissue Bank at Princess Margaret Cancer Centre/University Health Network with informed consent following procedures approved by the Research Ethics Board of the University Health Network (UHN REB 01-0573). AML samples were used as they have a relative high amount of cfDNA, allowing us to have 30 ng of cfDNA to divide into three technical replicates. Each researcher performed the cfMeDIP-seq method as per Shen et al. (2018),[2] with some minor changes. It was the aim to emulate batch effects that would be commonly seen in publicly available data from different labs for different studies. As such, researchers 1 and 3 used the same UMI as previous analyses. Researcher 2 used a 2 bp degenerate UMI.[28] For the ligation of the adapters, researchers 1 and 2 did an overnight incubation at 4 °C, while researcher 3 did a 2 h incubation at 20 °C. The number of PCR cycles to amplify the final library also differed between the batches. Researcher 1 ran 15 cycles, researcher 2 ran 13 cycles, and researcher 3 ran 11 cycles. Researchers 1 and 3 used antibody 1 (Diagenode, Denville, NJ, USA, Cat# C15200081-100, Lot# RD004, RRID: AB_2572207), while researcher 2 used antibody 2 (Diagenode, Denville, NJ, USA, Cat# C15200081-100, Lot# RD001, RRID: AB_2572207). AML samples were run on Illumina NovaSeq 6000 (Illumina, San Diego, CA, USA), paired-end 2×100 bp, 60 million reads per sample. A Gaussian generalized linear model was used to calculate molar amount and adjust for fragment length, G+C content, and CpG fraction in each batch independently. It was assessed whether spike-in controls mitigate batch effects by performing principal component analysis (PCA) on samples for which we calculated molar amount. PCA also performed on the same samples using only read counts as well as read counts preprocessed using QSEA,[29] the current standard processing pipeline of MeDIP-seq data, without the use of our spike-in controls. To investigate if known variables associate with each of the principal components, two-way ANOVA was performed between each principal component and each categorical variable. The categorical variables included: batch, sequencing machine, adapters, samples, and sex (inferred by Y chromosome signal). The resulting F-statistic was converted to an effect size, Cohen's *d,* using the compute.es package in R version 3.4.1.[30] P-values for multiple test correction was adjusted using the Holm-Bonferroni method.[31]

## Results 1

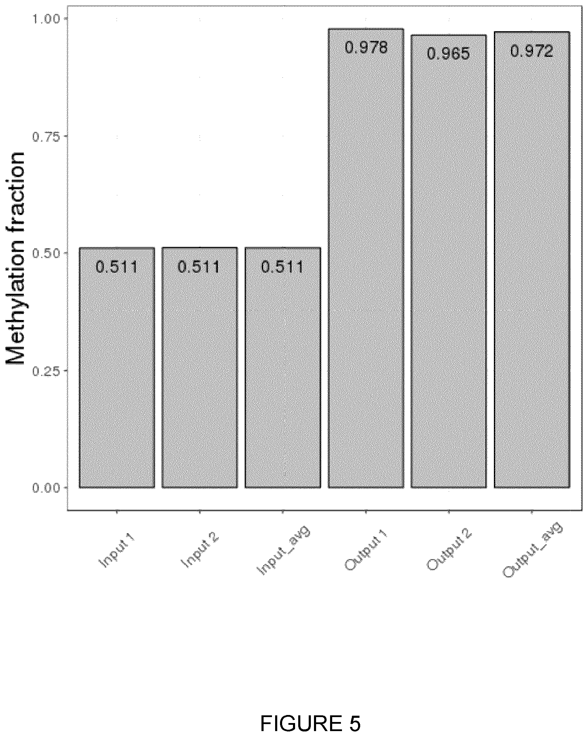

*Pilot testing.* On average, 51% of the input fragments were methylated and 49% were unmethylated. After undergoing cfMeDIP, on average, 97% of the fragments were methylated and 3% were unmethylated, representing unspecific binding (Figure 5). This is in concordance with with Shen et al. [6] which showed similar unspecific binding with qPCR validation. The enrichment for methylated sequences further supports the validity of the cfMeDIP-seq method.

To assess amplification bias towards particular fragment lengths, G+C content or number of CpGs, read count distributions were plotted for unique reads (deduplicated) for input and output samples by methylation status. The methylated fragments showed an enrichment of 160 bp fragments which were expect due to size selection step for fragments between 150-200 bp. Preference to higher G+C content was seen. After the cfMeDIP method, the enrichment for the 160 bp fragments was maintained and an enrichment was observed towards fragments with higher G+C content. No pattern appeared in the number of CpGs per fragment that influenced the fragments enriched for after the cfMeDIP protocol (Figure 6).

The unmethylated fragments showed the same enrichment to 160 bp fragments and higher G+C content. This suggested that the unspecific binding of the cfMeDIP method was partial to fragments with higher G+C content. There was no association between the number of CpGs present within a fragment and the number of reads (Figure 7).

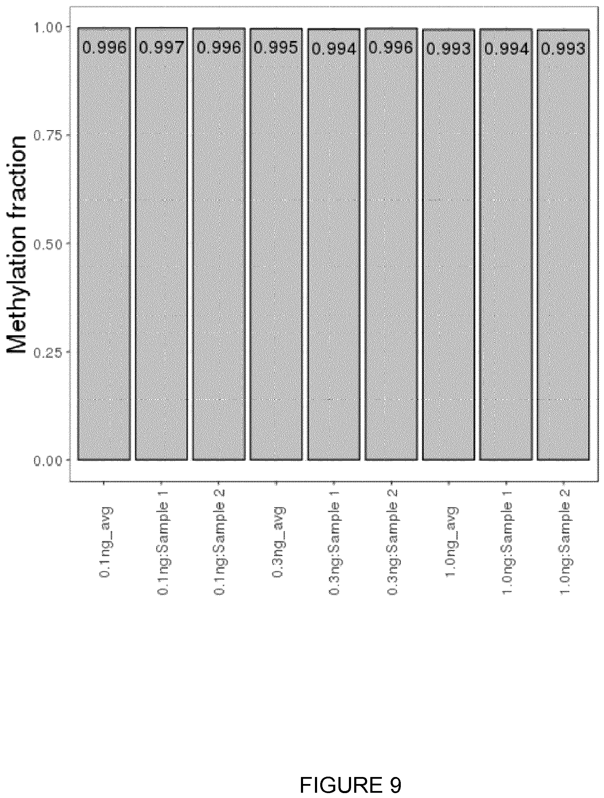

*Testing spike-in controls to HCT116.* With the addition of the cell line DNA, 99.9% specificity was achieved to 5-methylcytosine, with $\leq 0.1\%$ unspecific binding to non-methylated fragments (Figure 9). Similar to the pilot testing on only the synthetic fragments, a PCR bias effect was observed towards smaller fragments, which can be mitigated with using UMI barcodes to identify which fragments are PCR duplicates. Fragments were enriched for 160 bp, as per the size selection step for fragments between 150-200 bp. There was also an enrichment towards fragments with higher G+C content. These patterns were observed at each concentration we used for the spike-in controls.

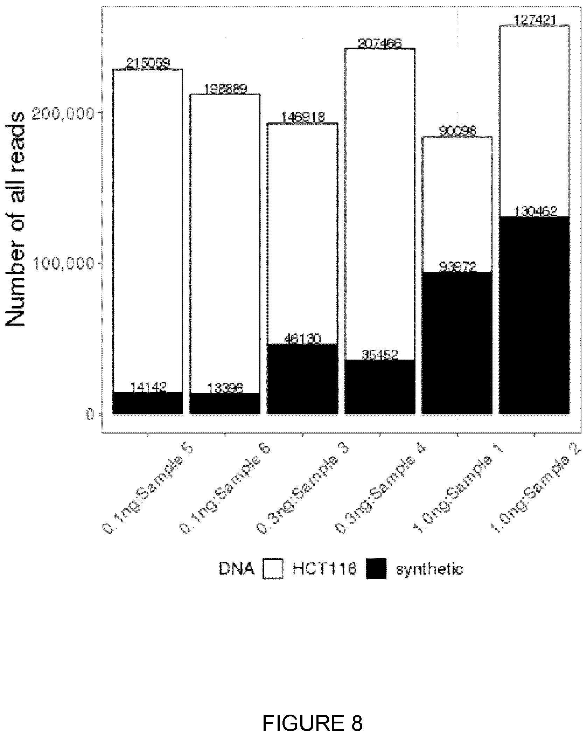

Number of reads being used on the spike-in controls at each molality were compared to number of reads being used by HCT116 is shown in Figure 8. Even at 0.1 ng of synthetic DNA spiked into 10 ng of HCT116, approximately 6% of the reads were used to the controls. Therefore, at higher resolution sequencing with 60 million reads per sample, 4 million reads would be used for spike-in controls in an experiment. This was more than needed to correct for batch effect. For this reason, lower amounts of spike-in controls were tested.

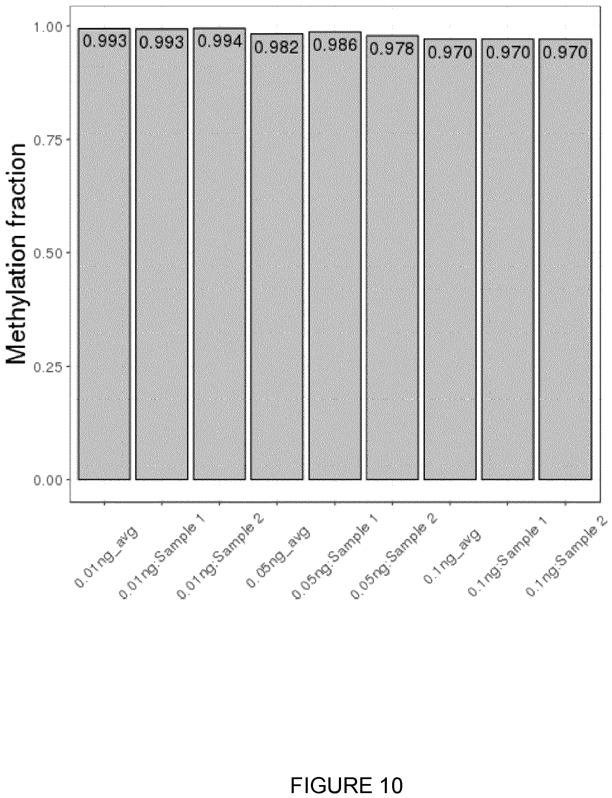

*Optimizing input concentration of spike-in controls.* The cfMeDIP-seq experiment enriched for methylated DNA >9.99% with ≤0.01% unspecific binding to non-methylated fragments (Figure 10). An enrichment was also observed for 160 bp fragments and higher G+C content. No patterns with the number of CpGs present in a fragment and read count was observed.

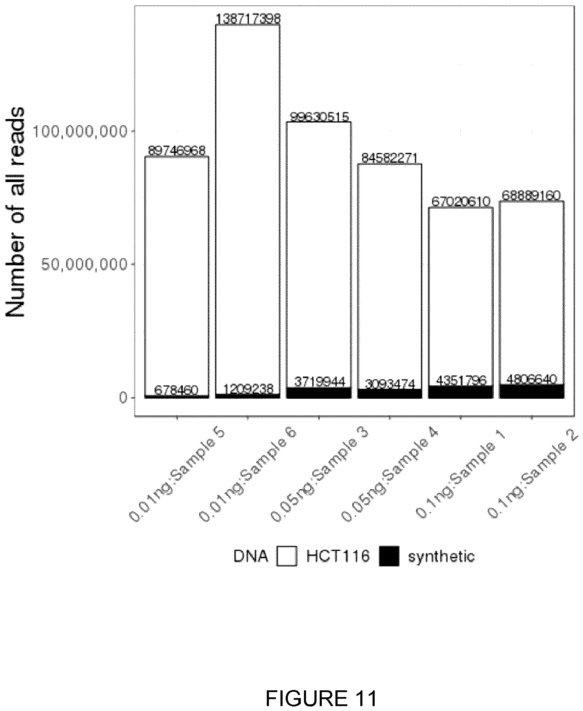

The total number of reads that were used towards the synthetic spike-in controls compared to the total number of reads used to our biological sample, HCT116 was assessed. This allowed optimization of the amount of spike-in controls that would be used in subsequent experiments, to maximize reads of a biological sample of interest while still getting enough information on the control fragments to correct biological and technical bias. Spiking in 0.01 ng of synthetic controls allowed use of ≤0.01% of the reads to the controls, while leaving the rest to the biological sample. There was still >650,000 reads of control sequence to use for analysis (Figure 11). Therefore, it was decided to use 0.01 ng of spike-in controls fragments in subsequent experiments.

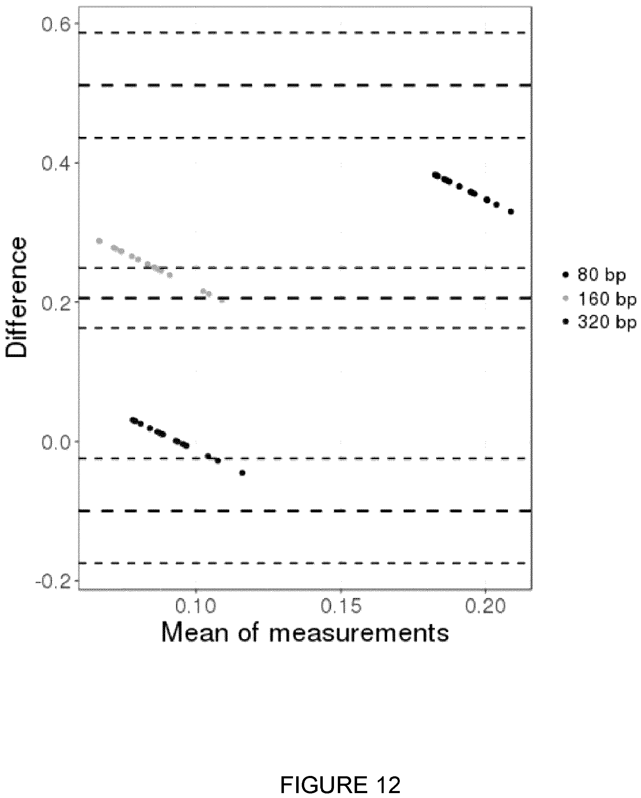

*Calculating concentration for spike-in controls.* The normalized fragment length and number of CpGs within a fragment were used, along with G + C content, and read count to model concentration (fmol/ng) (see Methods). Using a Gaussian generalized linear model with log link enabled estimation of concentration well ($r^2$=0.999) (Figure 12). The 80 bp fragments, performed less than the 160 bp and 320 bp fragments. However, as size selection was performed for 150-200 bp, this matters less for model performance under practical conditions.

## Results 2

*cfMeDIP-seq preferentially enriches for high G+C content regions.* When performing cfMeDIP-seq directly on the synthetic spike-in controls as the input sample (Figure 13), we observed 51% of the input fragments methylated and 49% unmethylated. After fragments underwent cfMeDIP, a shift in the fragment abundance was observed, with 97% of the sequenced reads corresponding to the methylated fragments. The enrichment for methylated sequences further supports the validity of the cfMeDIP-seq method.

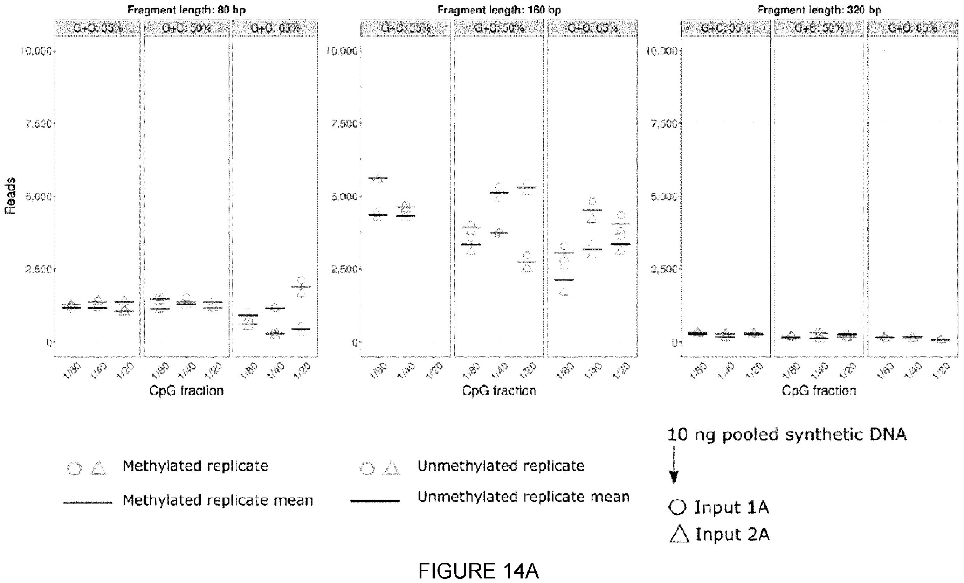

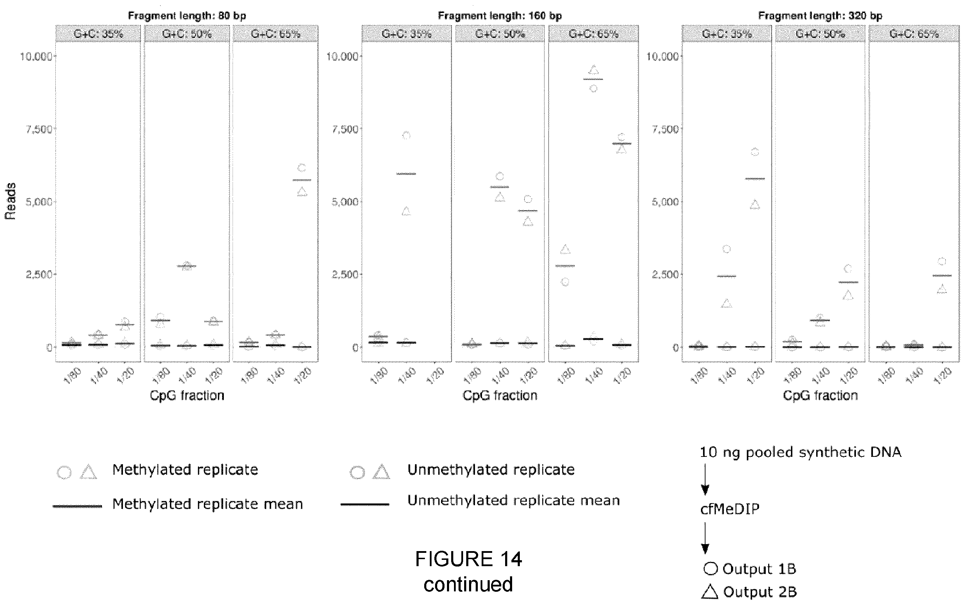

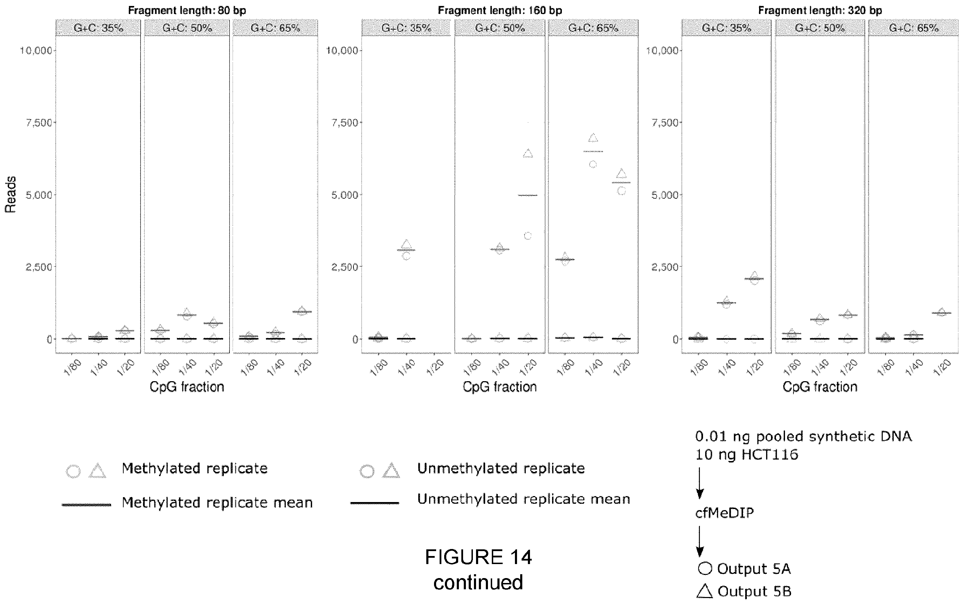

After cfMeDIP-seq, the synthetic spike-in controls output as well as the spike-in controls in 10 ng of HCT116 show an enrichment of 160 bp fragments which we expect due to our size selection step for fragments between 150 bp-200 bp. After the cfMeDIP method, we maintained enrichment for the 160 bp fragments and observe an enrichment towards fragments with higher G+C content and high CpG fraction (Figure 14).

Signal from unmethylated fragments for both the synthetic spike-in control output and 0.01 ng spike-in is not associated with fragment lengths, G+C content or CpG fraction (Figure 14). This suggests that the non-specific binding of the cfMeDIP method is random.

*Low input spike-in control proves sufficient to account for biological and technical variance.* The cfMeDIP-seq experiment using 0.01 ng of spike-in control DNA into 10 ng of HCT116 genomic DNA enriched for methylated DNA ≥99.99% with ≤0.01% non-specific binding to non-methylated fragments (Figure 14). An enrichment for 160 bp fragments and higher G+C content was also observed. Weaker signal in the fragments that have a CpG present in 1/80 bp was observed.

The total number of reads used towards spike-in controls was assessed compared to the total number of reads used towards the biological sample, HCT116 genomic DNA. This allowed optimization of the amount of spike-in controls to be used in subsequent experiments, maximizing reads to biological sample of interest while obtaining sufficient reads from the spike-in controls to correct for biological and technical bias. Spiking in 0.01 ng of synthetic spike-in control DNA into present cfMeDIP-seq experiments allowed use of ≤1% of the reads to the controls, while leaving the rest to biological sample. There were still >650,000 reads of control sequence to use for analysis. Therefore, it was decided to use 0.01 ng of spike-in control fragments in subsequent experiments.

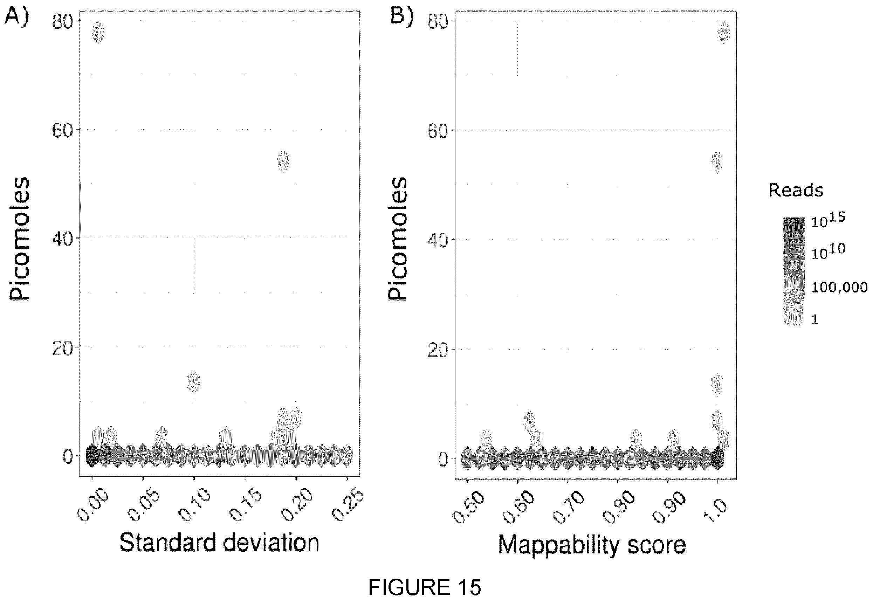

*Filtering problematic regions removes potential sources of biological and technical artifacts.* After filtering regions containing simple repeats, ENCODE blacklist regions, regions with mappability scores ≤ 0.5 and regions where standard deviation between the replicates ≥ 0.25, we observed no relationship between molar amount and standard deviation, and no relationship between molar amount and mappability scores. This suggests that removing these regions is beneficial to reduce biological and technical artifacts. There are 11 outlier windows as shown in Figure 15. These regions all had ≥ 2 pmol, shown in table 4.

**Table 4. 300 bp genomic windows with predicted molar amount ≥ 2 pmol.**

| Chromosome | Start[a] | End[a] | Amount | Element[b] | Family[b] | Name[b] |
|---|---|---|---|---|---|---|
| 13 | 95,176,201 | 95,176,500 | 78.15 pmol | SINE | Alu | AluJo |
| 2 | 120,383,401 | 120,383,700 | 55.01 pmol | SINE | MIR | MIR_Amn |
| 12 | 95,476,201 | 95,476,500 | 11.70 pmol | SINE | Alu | AluSx |
| 22 | 20,900,401 | 20,900,700 | 6.30 pmol | SINE | Alu | AluJb, AluY |
| 17 | 1,025,101 | 1,025,400 | 5.33 pmol | SINE | Alu | AluYe5, AluSx1 |
| X | 44,613,001 | 44,613,300 | 4.23 pmol | SINE | Alu | AluSp, AluJr |
| 1 | 44,582,701 | 44,583,000 | 3.80 pmol | SINE | Alu | AluSx1 |
| 8 | 139,704,301 | 139,704,600 | 3.74 pmol | Low complexity | - | G-rich |
| 2 | 224,230,501 | 224,230,800 | 2.49 pmol | LTR | ERV1 | HERVH-int |
| 17 | 3,521,101 | 3,521,400 | 2.45 pmol | LINE, DNA transposon | L2, hAT-Blackjack | L2b, MER63D |
| 16 | 11,578,801 | 11,579,100 | 2.36 pmol | LINE, SINE | L1, Alu | L1MD3, AluSp |

a Genomic position defined by hg38, 1-start, fully closed.

b All elements, families, and names that overlap our 300 bp genomic windows. Element, family, and name as defined in the UCSC Genome Browser RepeatMasker track, from RepeatMasker version 3.0.

All of these 11 "outlier" windows are repetitive elements, predominantly SINE elements (N = 8), mostly from the Alu family, whose origin traces back to primates. A HOMER analysis yielded no significant motifs.[26]

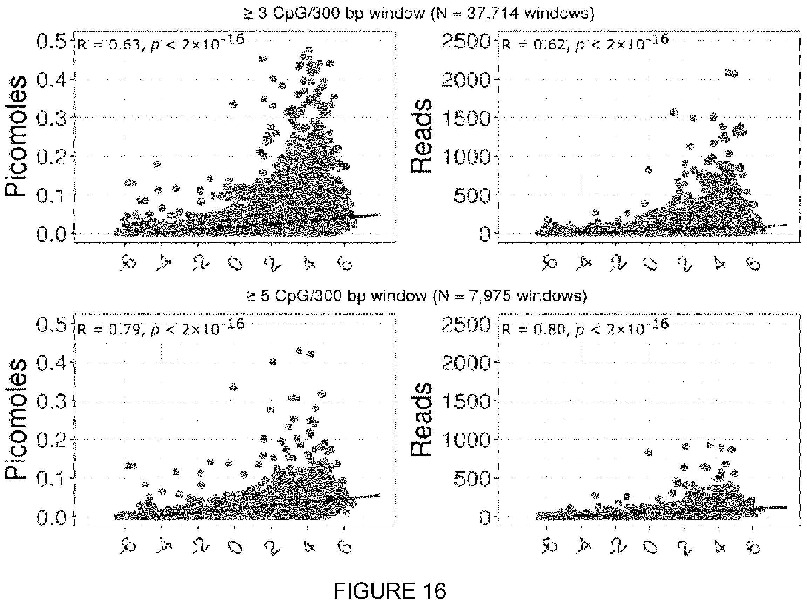

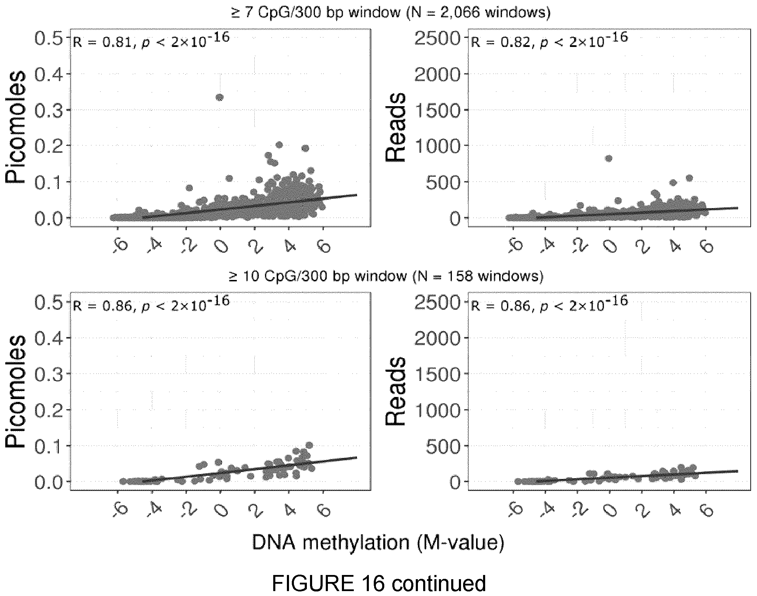

*Absolute quantification correlates with M-values and performs comparatively to read counts.* The linear model was used to calculate molar amount for each 300 bp genomic window. Significant correlation was observed between molar amount and M-values across the genome in our HCT116 genomic DNA samples. A higher correlation was observed when the analyses was restricted to high CpG dense regions, defined as 300 bp windows with ≥5 CpG probes representing DNA methylation on the EPIC array, within the 300 bp window. This is not surprising as the cfMeDIP-seq technique preferentially measures DNA methylation at high CpG dense regions. To compare with the current standard, read counts were correlated to M-values (Figure 16). It was shown that molar amount performs similarly to read counts, but has the advantage of allowing for absolute quantification.

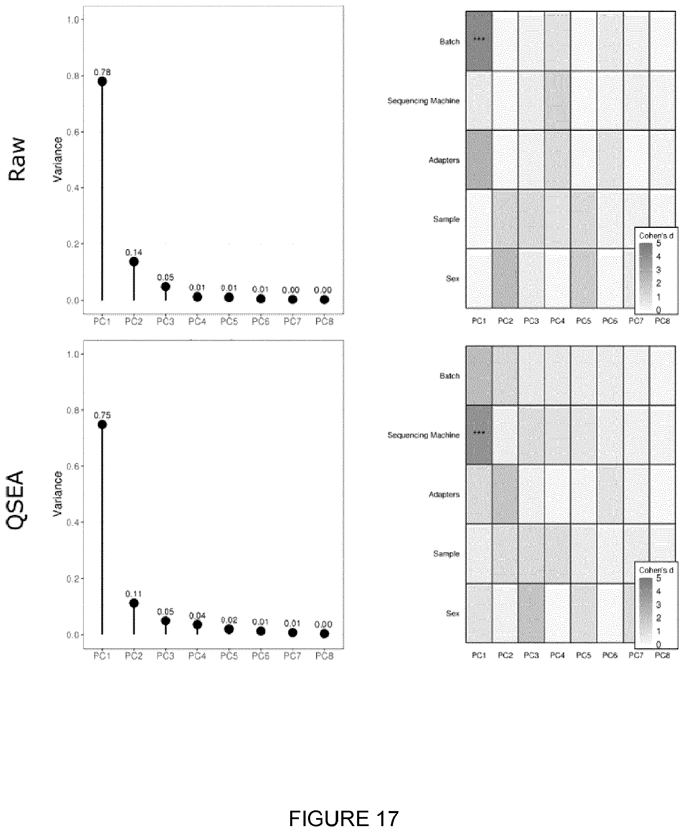

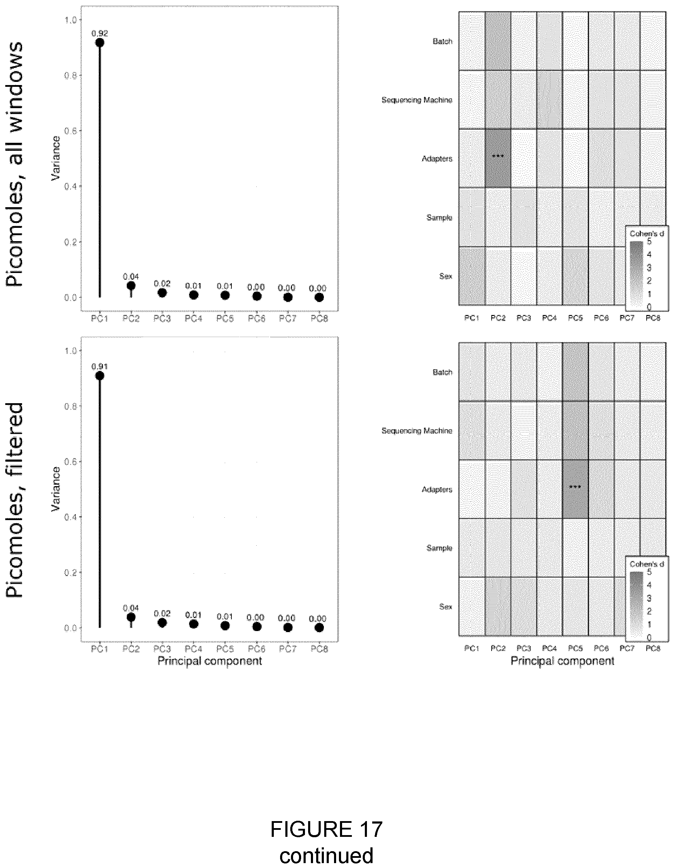

*Spike-in controls mitigate batch effects.* PCA on raw data, measured in read counts, showed that principal component 1, comprising of 76% of the variance is associated with processing batch (Figure 17). After QSEA normalization, principal component 1 is still associated with batch, although not significantly after multiple test correction, and sequencing machine is now significantly associated with principal component 1. This suggested that QSEA normalization may actually introduce variance into the data that is not biologically meaningful. Using molar amount measure greatly improved batch effects. Normalization of the data based on molar amounts in picomoles, generated without the application of any genomic filtering resulted in a shift of the batch processing variable to principal component 2, making up ≤5% of the variance (Figure 5). The addition of suggested filtering for regions containing simple repeats, ENOCDE blacklist regions and regions with low mappability, a further shirt of the batch processing effect to principal component 5 was seen, making up 1% of the variance (Figure 17). Further investigation into principal component 5, looking at the top 10% of windows driving this variance, 72% of these top regions are repetitive elements as defined by repeat masker,[33] predominantly Alu elements.

## Discussion

The data showed the validity of using synthetic spike-in control DNA to improve results of cfMeDIP-seq experiments. The difference in non-specific binding to 5-methylcytosine in experiment 1, cfMeDIP-seq directly on spike-in controls, and experiment 2, cfMeDIP-seq on 0.01 ng spiked into HCT116 genomic DNA, can likely be explained by the difference in the proportion of methylated CpGs in the experimental samples. The spike-in controls contained 51% methylated CpGs, while the human genome contains approximately 70% methylated CpGs.[34] To reduce technical and biological artifacts, it was important to filter potentially problematic regions prior to analysis. It was shown that removing regions with simple repeats, regions that overlap the ENCODE blacklist, regions with low mappability scores, and regions with high standard deviation between replicates helps to reduce outliers as well as technical and biological artifacts. It was shown that biological and technical bias exist in the cfMeDIP-seq data, and that the use of our spike-in controls helps to mitigate these biases.

Despite stringent filtering of potentially problematic regions, there were still outlier regions, which consisted mostly of repetitive elements, predominantly SINE elements While, these regions are CpG dense, our spike-in controls adjust for CpG fraction. Therefore, it is unlikely that high CpG density is the reason these regions are outliers. Interestingly, majority of the Alu outliers are the older Alu elements.[32] No specific transcription factor binding motifs are associated with these elements. It is possible, that because these elements are highly methylated,[32] it's likely overrepresentation of these regions were seen. Depending on the experimental question, some may choose to remove repetitive elements, such as LINEs and SINEs, from the analysis, in addition to previous recommendations for filtering. However, given that these repetitive elements take up relatively few windows, they are unlikely to drastically impact results.

It is shown that the use of spike-in controls helps to mitigate differences between batches due to a number of technical factors including: technician, adapters, sequencing machine, and adapter ligation incubation. Using spike-in controls it was observed that principal components significantly associated with batch made up ≤5% of the variance within the data. Whereas in raw data, or data normalized using QSEA had principal components significantly associated with processing batch contributing to ≥85% of the variance within the data.

This study gives strong evidence towards the beneficial impact of using spike-in controls to account for both biological and technical biases in MeDIP-seq experiments. Not only will it improve results within a given study, having these controls as gold standard will improve reproducibility across data generated from many labs.

## References 1

[1] Stephen F Altschul et al. "Basic local alignment search tool". In: Journal of molecular biology 215.3 (1990), pp. 403-410.

[2] Shifu Chen et al. "fastp: an ultra-fast all-in-one FASTQ preprocessor". In: Bioinformatics 34.17 (2018), pp. i884-i890.

[3] Ben Langmead and Steven L Salzberg. "Fast gapped-read alignment with Bowtie 2". In: Nature methods 9.4 (2012), p. 357.

[4] Richard Owczarzy et al. "IDT SciTools: a suite for analysis and design of nucleic acid oligomers". In: Nucleic acids research 36.suppl_2 (2008), W163-W169.

[5] Yann Ponty, Michel Termier, and Alain Denise. "GenRGenS: software for generating random genomic sequences and structures". In: Bioinformatics 22.12 (2006), pp. 1534-1535.

[6] Shu Yi Shen et al. "Sensitive tumour detection and classification using plasma cell-free DNA methylomes". In: Nature 563.7732 (2018), p. 579.

[7] Shu Yi Shen et al. "Preparation of cfMeDIP-seq libraries for methylome profiling of plasma cell-free DNA". In: Nature protocols 14.10 (2019), pp. 2749-2780.

[8] Zhenjiang Zech Xu and David H Mathews. "Secondary structure prediction of single sequences using RNAstructure". In: RNA Structure Determination. Springer, 2016, pp. 15-34.

## References 2

1. Shen, S. Y., Singhania, R., Fehringer, G., Chakravarthy, A., Roehrl, M. H., Chadwick, D., Zuzarte, P. C., Borgida, A., Wang, T. T., Li, T., et al. Sensitive tumour detection and classification using plasma cell-free DNA methylomes.

Nature 563, 579 (2018).

2. Shen, S. Y., Burgener, J. M., Bratman, S. V. & De Carvalho, D. D. Preparation of cfMeDIP-seq libraries for methylome profiling of plasma cell-free DNA. Nature protocols 14, 2749-2780 (2019).

3. Cao, F., Wei, A., Hu, X., He, Y., Zhang, J., Xia, L., Tu, K., Yuan, J., Guo, Z., Liu, H., et al. Integrated epigenetic biomarkers in circulating cell-free DNA as a robust classifier for pancreatic cancer. Clinical Epigenetics 12, 1-14 (2020).

4. Lasseter, K., Nassar, A. H., Hamieh, L., Berchuck, J. E., Nuzzo, P. V., Korthauer, K., Shinagare, A. B., Ogorek, B., McKay, R., Thorner, A. R., et al. Plasma cell-free DNA variant analysis compared with methylated DNA analysis in renal cell carcinoma. Genetics in Medicine, 1-8 (2020).

5. Nassiri, F., Chakravarthy, A., Feng, S., Shen, S. Y., Nejad, R., Zuccato, J. A., Voisin, M. R., Patil, V., Horbinski, C., Aldape, K., et al. Detection and discrimination of intracranial tumors using plasma cell-free DNA methylomes. Nature Medicine 26, 1044-1047 (2020).

6. Nuzzo, P. V., Berchuck, J. E., Korthauer, K., Spisak, S., Nassar, A. H., Abou Alaiwi, S., Chakravarthy, A., Shen, S. Y., Bakouny, Z., Boccardo, F., et al. Detection of renal cell carcinoma using plasma and urine cell-free DNA methylomes. Nature Medicine 26, 1041-1043 (2020).

7. Jiang, L., Schlesinger, F., Davis, C. A., Zhang, Y., Li, R., Salit, M., Gingeras, T. R. & Oliver, B. Synthetic spike-in standards for RNA-seq experiments. Genome research 21, 1543-1551 (2011).

8. Chen, K., Hu, Z., Xia, Z., Zhao, D., Li, W. & Tyler, J. K. The overlooked fact: fundamental need for spike-in control for virtually all genome-wide analyses. Molecular and cellular biology 36, 662-667 (2016).

9. Orlando, D. A., Chen, M. W., Brown, V. E., Solanki, S., Choi, Y. J., Olson, E. R., Fritz, C. C., Bradner, J. E. & Guenther, M. G. Quantitative ChIP-Seq normalization reveals global modulation of the epigenome. Cell reports 9, 1163-1170 (2014).

10. Deveson, I. W., Chen, W. Y., Wong, T., Hardwick, S. A., Andersen, S. B., Nielsen, L. K., Mattick, J. S. & Mercer, T. R. Representing genetic variation with synthetic DNA standards. Nature methods 13, 784 (2016).

11. Blackburn, J., Wong, T., Madala, B. S., Barker, C., Hardwick, S. A., Reis, A. L., Deveson, I. W. & Mercer, T. R. Use of synthetic DNA spike-in controls (sequins) for human genome sequencing. Nature Protocols 14, 2119 (2019).

12. Mouliere, F., Chandrananda, D., Piskorz, A. M., Moore, E. K., Morris, J., Ahlborn, L. B., Mair, R., Goranova, T., Marass, F., Heider, K., et al. Enhanced detection of circulating tumor DNA by fragment size analysis. Science translational medicine 10 (2018).

13. Ponty, Y., Termier, M. & Denise, A. GenRGenS: software for generating random genomic sequences and structures. Bioinformatics 22, 1534-1535 (2006).

14. Altschul, S. F., Gish, W., Miller, W., Myers, E. W. & Lipman, D. J. Basic local alignment search tool. Journal of molecular biology 215, 403-410 (1990).

15. Graves-Lindsay, T., Albracht, D., Fulton, R. S., Kremitzki, M., Magrini, V., Markovic, C., McGrath, S., Steinberg, K. M., Wilson, R. K., et al. Evaluation of GRCh38 and de novo haploid genome assemblies demonstrates the enduring quality of the reference assembly. Genome research 27, 849-864 (2017).

16. Owczarzy, R., Tataurov, A. V., Wu, Y., Manthey, J. A., McQuisten, K. A., Almabrazi, H. G., Pedersen, K. F., Lin, Y., Garretson, J., McEntaggart, N. O., et al. IDT SciTools: a suite for analysis and design of nucleic acid oligomers. Nucleic acids research 36, W163-W169 (2008).

17. Xu, Z. Z. & Mathews, D. H. Secondary structure prediction of single sequences using RNA structure. RNA structure determination, 15-34 (Springer, 2016).

18. Chen, S., Zhou, Y., Chen, Y. & Gu, J. fastp: an ultra-fast all-in-one FASTQ preprocessor. Bioinformatics 34, i884-i890 (2018).

19. Ewing, B. & Green, P. Base-calling of automated sequencer traces using phred. II. Error probabilities. Genome research 8, 186-194 (1998).

20. Langmead, B. & Salzberg, S. L. Fast gapped-read alignment with Bowtie 2. Nature methods 9, 357 (2012).

21. R Core Team. R: A Language and Environment for Statistical Computing R Foundation for Statistical Computing (Vienna, Austria, 2013).

22. Quinlan, A. R. & Hall, I. M. BEDTools: a flexible suite of utilities for comparing genomic features. Bioinformatics 26, 841-842 (2010).

23. Karimzadeh, M., Ernst, C., Kundaje, A. & Hoffman, M. M. Umap and Bismap: quantifying genome and methylome mappability. Nucleic acids research 46, e120 (2018).

24. Karolchik, D., Hinrichs, A. S., Furey, T. S., Roskin, K. M., Sugnet, C. W., Haussler, D. & Kent, W. J. The UCSC Table Browser data retrieval tool. Nucleic acids research 32, D493-D496 (2004).

25. Amemiya, H. M., Kundaje, A. & Boyle, A. P. The ENCODE blacklist: identification of problematic regions of the genome. Scientific reports 9, 1-5 (2019).

26. Heinz, S., Benner, C., Spann, N., Bertolino, E., Lin, Y. C., Laslo, P., Cheng, J. X., Murre, C., Singh, H. & Glass, C. K. Simple combinations of lineage-determining transcription factors prime cis-regulatory elements required for macrophage and B cell identities. Molecular cell 38, 576-589 (2010).

27. Zhou, W., Triche Jr, T. J., Laird, P. W. & Shen, H. SeSAMe: reducing artifactual detection of DNA methylation by Infinium BeadChips in genomic deletions. Nucleic acids research 46, e123-e123 (2018).

28. Wang, T. T., Abelson, S., Zou, J., Li, T., Zhao, Z., Dick, J. E., Shlush, L. I., Pugh, T. J. & Bratman, S. V. High efficiency error suppression for accurate detection of low-frequency variants. Nucleic acids research 47, e87-e87 (2019).

29. Lienhard, M., Grasse, S., Rolff, J., Frese, S., Schirmer, U., Becker, M., Börno, S., Timmermann, B., Chavez, L., Sültmann, H., et al. QSEA-modelling of genome-wide DNA methylation from sequencing enrichment experiments. Nucleic acids research 45, e44-e44 (2017).

30. Re, A. C. D. compute.es: Compute Effect Sizes (2013).

31. Holm, S. A simple sequentially rejective multiple test procedure. Scandinavian journal of statistics, 65-70 (1979).

32. Deininger, P. Alu elements: know the SINEs. Genome biology 12, 236 (2011).

33. Tarailo-Graovac, M. & Chen, N. Using RepeatMasker to identify repetitive elements in genomic sequences. Current protocols in bioinformatics 25, 4-10 (2009).

34. Strichman-Almashanu, L. Z., Lee, R. S., Onyango, P. O., Perlman, E., Flam, F., Frieman, M. B. & Feinberg, A. P. A genome-wide screen for normally methylated human CpG islands that can identify novel imprinted genes. Genome research 12, 543-554 (2002).

The invention includes the aspects in the following paragraphs

1. A method of capturing and analyzing cell-free methylated DNA in a sample, the method comprising the steps of:

    a. subjecting the sample to library preparation to permit subsequent sequencing of the cell-free methylated DNA;

    b. adding a predetermined amount of a set of control synthetic DNA fragments, wherein the control synthetic DNA fragments each have a known nucleic acid sequence that does not substantially align to a target genome sequence, and wherein at least some of the control synthetic DNA fragments in the set are methylated;

c. denaturing the sample;

d. capturing cell-free methylated DNA and the control synthetic DNA fragments using a binder selective for methylated polynucleotides; and

e. amplifying and sequencing the captured cell-free methylated DNA and the control synthetic DNA fragments.

2. The method of paragraph 1, further comprising:
f. calculating an amount, a concentration, or a molality of the cell-free methylated DNA in the sample based on the sequenced control synthetic DNA fragments.

3. The method of paragraph 1 or 2, wherein the set of control synthetic DNA fragments comprises between 3 to 7 predetermined fragment lengths.

4. The method of paragraph 3, wherein the set of control synthetic DNA fragments comprises 3 predetermined fragment lengths.

5. The method of any one of paragraphs 1-3, wherein the control synthetic DNA fragments are 50 to 500 base pairs (bp) in length, preferably 80 to 320 bp in length.

6. The method of paragraph 4, wherein the three predetermined fragment lengths are 80 bp, 160 bp, and 320 bp, respectively.

7. The method of any one of paragraphs 1-6, wherein the control synthetic DNA fragments have a combined guanine and cytosine (G+C) content of between 0% to 100%, preferably between 25% to 75%.

8. The method of paragraph 4, wherein the three predetermined fragment lengths have a G+C content of 35%, 50%, and 65%, respectively.

9. The method of paragraph 1, wherein each of the control synthetic DNA fragments comprises a number of CpG dinucleotides ranging between 0 and ½ of the length of the fragment in base pairs.

10. The method of paragraph 8, wherein each of the control synthetic DNA fragments comprises 1 to 25 CpG dinucleotides, preferably 1 to 16 CpG dinucleotides.

11. The method of paragraph 1, wherein the control synthetic DNA fragments have a nucleic acid sequence as set forth in on or more of SEQ ID NO: 1-59.

12. The method of paragraph 1, further comprising:

i. sequencing the captured cell-free methylated DNA and the control synthetic DNA fragments;

ii. comparing the sequenced cell-free methylated DNA against the known nucleic acid sequences of the control synthetic DNA fragments; and

iii. comparing any unmatched DNA from (ii) against the target genome sequence.

13. The method of any one of paragraphs 1 to 12, wherein half of the control synthetic DNA fragments in the set are methylated, and the other half are unmethylated.

14. The method of any one of paragraphs 1 to 13, wherein the set of control synthetic DNA fragments comprise a first methylated sequence and a second unmethylated sequence.

15. The method of any one of paragraphs 1 to 12, wherein all of the control synthetic DNA fragments in the set are methylated.

16. The method of paragraph 2, comprising estimating the amount of captured cell-free methylated DNA before amplification using unique molecular identifier (UMI) adapters.

17. The method of any one of paragraphs 1-16, wherein the binder is a protein comprising a methyl-CpG-binding domain.

18. The method of paragraph 17, wherein the protein is a MBD2 protein.

19. The method of any one of paragraphs 1-18, wherein step (d) comprises immunoprecipitating the cell-free methylated DNA using an antibody.

20. The method of paragraph 19, comprising adding at least 0.05 $\mu$g of the antibody to the sample for immunoprecipitation, and preferably at least 0.16 $\mu$g.

21. The method of paragraph 20, wherein the antibody is 5-methylcytosine antibody, 5-hydroxymethylcytosine antibody, 5-formylcytosine antibody, or 5-carboxylcytosine antibody.

22. The method of any one of paragraphs 1-21, wherein the sample has less than 100 ng of cell-free DNA, and the method further comprises adding a first amount of filler DNA to the sample, wherein at least a portion of the filler DNA is methylated.

23. The method of paragraph 22, wherein the first amount of filler DNA comprises about 5%, 10%, 15%, 20%, 30%, 40%, 50%, 60%, 70%, 80%, 90%, or 100% methylated filler DNA with remainder being unmethylated filler DNA, and preferably between 5% and 50%, between 10%-40%, or between 15%-30% methylated filler DNA.

24. The method of paragraph 22, wherein the first amount of filler DNA is from 20 ng to 100 ng, preferably 30 ng to 100 ng, more preferably 50 ng to 100 ng.

25. The method of paragraph 22, wherein the cell-free DNA from the sample and the first amount of filler DNA together comprises at least 50 ng of total DNA, preferably at least 100 ng of total DNA.

26. The method of paragraph 22, wherein the filler DNA is 50 bp to 800 bp long, preferably 100 bp to 600 bp long, and more preferably 200 bp to 600 bp long.

27. The method of paragraph 22, wherein the filler DNA is endogenous or exogenous DNA.

28. The method of paragraph 27, wherein the filler DNA is non-human DNA, preferably $\lambda$ DNA.

29. The method of paragraph 27, wherein the filler DNA has no alignment to human DNA.

30. A method of identifying a sequence for a control synthetic DNA fragment for use in capturing and analysis of cell-free methylated DNA, the method comprising the steps of:

    a. generating nucleic acid sequences based on a plurality of target fragment lengths, a target combined guanine and cytosine (G+C) content, and a target number of CpG dinucleotides for each fragment; and

    b. eliminating generated sequences that align to a human genome;

        wherein the plurality of target fragment lengths comprises 3 to 7 target fragment lengths between 50 to 500 base pairs (bp);

        wherein the target G+C content is between 0% to 100%; and

        wherein the target number of CpG dinucleotides for each fragment is between 0 and ½ of the length of the fragment in base pairs.

31. The method of paragraph 30, wherein the target fragment length is, preferably 80 to 320 bp.

32. The method of paragraph 31, wherein the target fragment lengths are multiples of a shortest fragment length.

33. The method of paragraph 32, wherein the plurality of target fragment lengths comprise three fragments of 80 bp, 160 bp, or 320 bp.

34. The method of paragraph 30, wherein the target G+C content is between 25% to 75%.

35. The method of paragraph 34, wherein the target G+C content is 35%, 50%, or 65%, respectively.

36. The method of paragraph 30, wherein the target number of CpG dinucleotides is 1-25, preferably 1-16 CpG dinucleotides per fragment.

37. The method of paragraph 30, wherein the target number of CpG dinucleotides is one per 20 bp, per 40 bp, or per 80 bp.

**Claims**

1. A method comprising:

   (a) generating a mixture comprising (1) a plurality of DNA molecules derived from the cell-free sample, (2) a plurality of control synthetic nucleic acid molecules and (3) a plurality of filler nucleic acid molecules, wherein at least a portion of the plurality of control synthetic nucleic acid molecules is methylated and at least a portion of the plurality of filler nucleic acid molecules is methylated;
   (b) using the plurality of filler nucleic acid molecules to enrich for methylated DNA molecules of the plurality of DNA molecules, thereby yielding a plurality of enriched DNA molecules; and
   (c) sequencing (i) the plurality of enriched DNA molecules or derivative thereof and (ii) the plurality of control synthetic nucleic acid molecules or derivative thereof to generate a plurality of sequencing reads comprising a first plurality of sequences of the plurality of enriched DNA molecules and a second plurality of sequences of the plurality of control synthetic nucleic acid molecules.

2. The method of claim 1, wherein the plurality of control synthetic nucleic acid molecules comprises at least two nucleic acid molecules comprising a sequence of a set of control sequences; wherein the set of control sequences comprises a plurality of target fragment lengths, a target combined guanine and cytosine (G+C) content, and a target number of CpG dinucleotides; optionally, wherein the set of control sequences do not substantially align to a human genome.

3. The method of claim 2, wherein the plurality of target fragment lengths comprises at least 3 predetermined fragment lengths; optionally, the target fragment lengths are from 50 to 500 base pairs (bp); further optionally, the target fragment lengths are from 80 to 320 bp.

4. The method of claim 2, wherein the target combined guanine and cytosine (G+C) content is between 25% to 75%.

5. The method of claim 2, wherein the target number of CpG dinucleotides is 1-25 per molecule.

6. The method of claim 2, further comprising: (d) calculating a concentration or amount of the cell-free methylated DNA in the sample based at least in part on the plurality of sequencing reads.

7. The method of claim 6, wherein the calculating in (d) comprises (i) generating a statistical model based at least in part on the second plurality of sequences and one or more of (1) the plurality of target fragment lengths, (2) the target G+C content, or (3) the target number of CpG dinucleotides; and (ii) using the statistical model to calculate the amount of concentration of the cell-free methylated DNA from the sample based at least in part on the first plurality of sequencing reads, and optionally wherein the statistical model comprises a generalized linear model (GLM).

8. The method of claim 1, wherein said first plurality of sequences comprises a bias and the method further comprises: (d) correcting said bias based at least in part on said second plurality of sequences.

9. The method of claim 1, wherein the plurality of control synthetic nucleic acid molecules comprise a first methylated sequence and a second unmethylated sequence, and optionally wherein all of the control synthetic DNA fragments in the set of control synthetic DNA fragments are at least partially methylated.

10. The method of claim 1, wherein (b) is performed using a binder that comprises a protein comprising a methyl-CpG-binding domain, optionally wherein the protein is a MBD2 protein or a functional variant thereof.

11. The method of claim 1, wherein (b) comprises immunoprecipitating the cell-free methylated DNA using an antibody, and optionally wherein the antibody is present in an amount of at least 0.05 micrograms.

12. The method of claim 11, wherein the antibody is a 5-methylcytosine antibody, a 5-hydroxymethylcytosine antibody, a 5-formylcytosine antibody, or a 5-carboxylcytosine antibody.

13. The method of claim 1, wherein the plurality of cell-free DNA molecules derived from the cell-free sample and an amount of the plurality of filler nucleic acid molecules together comprises at least 50 nanograms (ng) of total DNA.

14. The method of claim 1, wherein the plurality of filler nucleic acid molecules comprises at least about 15% methylated filler DNA and optionally wherein the plurality of filler nucleic acid molecules is present in an amount of 20 ng to 100 ng.

15. The method of claim 1, wherein the plurality of filler nucleic acid molecules is 50 to 800 bp long, optionally wherein the plurality of filler nucleic acid molecules is endogenous or exogenous DNA, and further optionally wherein the filler DNA is λ DNA.

FIGURE 1

FIGURE 2

(B.) FIGURE 3B

(A.) FIGURE 3A

(A.)

FIGURE 4A

(B.)

FIGURE 4B

FIGURE 5

FIGURE 6B

(B.)

FIGURE 6A

(A.)

FIGURE 6D

(D.)

FIGURE 6C

(C.)

(E.)

FIGURE 6E

(F.)

FIGURE 6F

(B.) FIGURE 7B

(A.) FIGURE 7A

(C.)

FIGURE 7C

(D.)

FIGURE 7D

(E.)

FIGURE 7E

(F.)

FIGURE 7F

FIGURE 8

FIGURE 9

FIGURE 10

FIGURE 11

FIGURE 12

FIGURE 13

48

FIGURE 13
continued

FIGURE 14A

FIGURE 14
continued

FIGURE 14
continued

FIGURE 15

FIGURE 16

FIGURE 16 continued

FIGURE 17

FIGURE 17
continued

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SONG et al.** *Trends Biochem Sci.,* October 2013, vol. 38 (10), 480-484
- **SHEN et al.** Samples underwent sequencing. Princess Margaret Genomics Centre, 2018
- **STEPHEN F ALTSCHUL et al.** Basic local alignment search tool. *Journal of molecular biology,* 1990, vol. 215 (3), 403-410
- **SHIFU CHEN et al.** fastp: an ultra-fast all-in-one FASTQ preprocessor. *Bioinformatics,* 2018, vol. 34 (17), i884-i890
- **BEN LANGMEAD ; STEVEN L SALZBERG.** Fast gapped-read alignment with Bowtie 2. *Nature methods,* 2012, vol. 9 (4), 357
- **RICHARD OWCZARZY et al.** IDT SciTools: a suite for analysis and design of nucleic acid oligomers. *Nucleic acids research,* 2008, vol. 36 (2), W163-W169
- **YANN PONTY ; MICHEL TERMIER ; ALAIN DENISE.** GenRGenS: software for generating random genomic sequences and structures. *Bioinformatics,* 2006, vol. 22 (12), 1534-1535
- **SHU YI SHEN et al.** Sensitive tumour detection and classification using plasma cell-free DNA methylomes. *Nature,* 2018, vol. 563 (7732), 579
- **SHU YI SHEN et al.** Preparation of cfMeDIP-seq libraries for methylome profiling of plasma cell-free DNA. *Nature protocols,* 2019, vol. 14 (10), 2749-2780
- Secondary structure prediction of single sequences using RNAstructure. **ZHENJIANG ZECH XU ; DAVID H MATHEWS.** RNA Structure Determination. Springer, 2016, 15-34
- **SHEN, S. Y. ; SINGHANIA, R. ; FEHRINGER, G. ; CHAKRAVARTHY, A. ; ROEHRL, M. H. ; CHADWICK, D. ; ZUZARTE, P. C. ; BORGIDA, A. ; WANG, T. T ; LI, T. et al.** Sensitive tumour detection and classification using plasma cell-free DNA methylomes. *Nature,* 2018, vol. 563, 579
- **SHEN, S. Y. ; BURGENER, J. M. ; BRATMAN, S. V. ; DE CARVALHO, D. D.** Preparation of cfMeDIP-seq libraries for methylome profiling of plasma cell-free DNA. *Nature protocols,* 2019, vol. 14, 2749-2780
- **CAO, F. ; WEI, A. ; HU, X. ; HE, Y. ; ZHANG, J. ; XIA, L. ; TU, K. ; YUAN, J. ; GUO, Z. ; LIU, H. et al.** Integrated epigenetic biomarkers in circulating cell-free DNA as a robust classifier for pancreatic cancer. *Clinical Epigenetics,* 2020, vol. 12, 1-14

- **LASSETER, K. ; NASSAR, A. H. ; HAMIEH, L. ; BERCHUCK, J. E. ; NUZZO, P. V. ; KORTHAUER, K. ; SHINAGARE, A. B. ; OGOREK, B. ; MCKAY, R. ; THORNER, A. R. et al.** Plasma cell-free DNA variant analysis compared with methylated DNA analysis in renal cell carcinoma. *Genetics in Medicine,* 2020, 1-8
- **NASSIRI, F. ; CHAKRAVARTHY, A. ; FENG, S. ; SHEN, S. Y. ; NEJAD, R. ; ZUCCATO, J. A. ; VOISIN, M. R. ; PATIL, V. ; HORBINSKI, C. ; ALDAPE, K. et al.** Detection and discrimination of intracranial tumors using plasma cell-free DNA methylomes. *Nature Medicine,* 2020, vol. 26, 1044-1047
- **NUZZO, P. V. ; BERCHUCK, J. E. ; KORTHAUER, K. ; SPISAK, S. ; NASSAR, A. H. ; ABOU ALAIWI, S. ; CHAKRAVARTHY, A. ; SHEN, S. Y. ; BAKOUNY, Z. ; BOCCARDO, F. et al.** Detection of renal cell carcinoma using plasma and urine cell-free DNA methylomes. *Nature Medicine,* 2020, vol. 26, 1041-1043
- **JIANG, L. ; SCHLESINGER, F. ; DAVIS, C. A. ; ZHANG, Y. ; LI, R. ; SALIT, M. ; GINGERAS, T. R. ; OLIVER, B.** Synthetic spike-in standards for RNA-seq experiments. *Genome research,* 2011, vol. 21, 1543-1551
- **CHEN, K. ; HU, Z. ; XIA, Z. ; ZHAO, D. ; LI, W. ; TYLER, J. K.** The overlooked fact: fundamental need for spike-in control for virtually all genome-wide analyses. *Molecular and cellular biology,* 2016, vol. 36, 662-667
- **ORLANDO, D. A. ; CHEN, M. W. ; BROWN, V. E. ; SOLANKI, S. ; CHOI, Y. J. ; OLSON, E. R. ; FRITZ, C. C. ; BRADNER, J. E. ; GUENTHER, M. G.** Quantitative ChIP-Seq normalization reveals global modulation of the epigenome. *Cell reports,* 2014, vol. 9, 1163-1170
- **DEVESON, I. W. ; CHEN, W. Y. ; WONG, T. ; HARDWICK, S. A. ; ANDERSEN, S. B. ; NIELSEN, L. K. ; MATTICK, J. S. ; MERCER, T. R.** Representing genetic variation with synthetic DNA standards. *Nature methods,* 2016, vol. 13, 784
- **BLACKBURN, J. ; WONG, T. ; MADALA, B. S. ; BARKER, C. ; HARDWICK, S. A. ; REIS, A. L. ; DEVESON, I. W. ; MERCER, T. R.** Use of synthetic DNA spike-in controls (sequins) for human genome sequencing. *Nature Protocols,* 2019, vol. 14, 2119

- **MOULIERE, F. ; CHANDRANANDA, D. ; PISKORZ, A. M. ; MOORE, E. K. ; MORRIS, J. ; AHLBORN, L. B. ; MAIR, R. ; GORANOVA, T. ; MARASS, F. ; HEIDER, K. et al.** Enhanced detection of circulating tumor DNA by fragment size analysis. *Science translational medicine,* 2018, vol. 10
- **PONTY, Y. ; TERMIER, M. ; DENISE, A.** GenRGenS: software for generating random genomic sequences and structures. *Bioinformatics,* 2006, vol. 22, 1534-1535
- **ALTSCHUL, S. F. ; GISH, W. ; MILLER, W. ; MYERS, E. W. ; LIPMAN, D. J.** Basic local alignment search tool. *Journal of molecular biology,* 1990, vol. 215, 403-410
- **GRAVES-LINDSAY, T. ; ALBRACHT, D. ; FULTON, R. S. ; KREMITZKI, M. ; MAGRINI, V. ; MARKOVIC, C. ; MCGRATH, S. ; STEINBERG, K. M. ; WILSON, R. K. et al.** Evaluation of GRCh38 and de novo haploid genome assemblies demonstrates the enduring quality of the reference assembly. *Genome research,* 2017, vol. 27, 849-864
- **OWCZARZY, R. ; TATAUROV, A. V. ; WU, Y. ; MANTHEY, J. A. ; MCQUISTEN, K. A. ; ALMABRAZI, H. G. ; PEDERSEN, K. F. ; LIN, Y. ; GARRETSON, J. ; MCENTAGGART, N. O. et al.** IDT SciTools: a suite for analysis and design of nucleic acid oligomers. *Nucleic acids research,* 2008, vol. 36, W163-W169
- Secondary structure prediction of single sequences using RNA structure. **XU, Z. Z. ; MATHEWS, D. H.** RNA structure determination. Springer, 2016, 15-34
- **CHEN, S. ; ZHOU, Y. ; CHEN, Y. ; GU, J.** fastp: an ultra-fast all-in-one FASTQ preprocessor. *Bioinformatics,* 2018, vol. 34, i884-i890
- **EWING, B. ; GREEN, P.** Base-calling of automated sequencer traces using phred. II. Error probabilities. *Genome research,* 1998, vol. 8, 186-194
- **LANGMEAD, B. ; SALZBERG, S. L.** Fast gapped-read alignment with Bowtie 2. *Nature methods,* 2012, vol. 9, 357
- *R: A Language and Environment for Statistical Computing R Foundation for Statistical Computing (Vienna, Austria,* 2013
- **QUINLAN, A. R. ; HALL, I. M.** BEDTools: a flexible suite of utilities for comparing genomic features. *Bioinformatics,* 2010, vol. 26, 841-842
- **KARIMZADEH, M. ; ERNST, C. ; KUNDAJE, A. ; HOFFMAN, M. M.** Umap and Bismap: quantifying genome and methylome mappability. *Nucleic acids research,* 2018, vol. 46, e120
- **KAROLCHIK, D. ; HINRICHS, A. S. ; FUREY, T. S. ; ROSKIN, K. M. ; SUGNET, C. W. ; HAUSSLER, D. ; KENT, W. J.** The UCSC Table Browser data retrieval tool. *Nucleic acids research,* 2004, vol. 32, D493-D496
- **AMEMIYA, H. M. ; KUNDAJE, A. ; BOYLE, A. P.** The ENCODE blacklist: identification of problematic regions of the genome. *Scientific reports,* 2019, vol. 9, 1-5
- **HEINZ, S. ; BENNER, C. ; SPANN, N. ; BERTOLINO, E. ; LIN, Y. C. ; LASLO, P. ; CHENG, J. X. ; MURRE, C. ; SINGH, H. ; GLASS, C. K.** Simple combinations of lineage-determining transcription factors prime cis-regulatory elements required for macrophage and B cell identities. *Molecular cell,* 2010, vol. 38, 576-589
- **ZHOU, W. ; TRICHE JR, T. J. ; LAIRD, P. W. ; SHEN, H.** SeSAMe: reducing artifactual detection of DNA methylation by Infinium BeadChips in genomic deletions. *Nucleic acids research,* 2018, vol. 46, e123-e123
- **WANG, T. T. ; ABELSON, S ; ZOU, J. ; LI, T. ; ZHAO, Z. ; DICK, J. E. ; SHLUSH, L. I. ; PUGH, T. J. ; BRATMAN, S. V.** High efficiency error suppression for accurate detection of low-frequency variants. *Nucleic acids research,* 2019, vol. 47, e87-e87
- **LIENHARD, M. ; GRASSE, S. ; ROLFF, J. ; FRESE, S. ; SCHIRMER, U. ; BECKER, M. ; BÖRNO, S. ; TIMMERMANN, B. ; CHAVEZ, L. ; SÜLTMANN, H. et al.** QSEA-modelling of genome-wide DNA methylation from sequencing enrichment experiments. *Nucleic acids research,* 2017, vol. 45, e44-e44
- **RE, A. C. D.** *compute.es: Compute Effect Sizes,* 2013
- **HOLM, S.** A simple sequentially rejective multiple test procedure. *Scandinavian journal of statistics,* 1979, 65-70
- **DEININGER, P.** Alu elements: know the SINEs. *Genome biology,* 2011, vol. 12, 236
- **TARAILO-GRAOVAC, M. ; CHEN, N.** Using RepeatMasker to identify repetitive elements in genomic sequences. *Current protocols in bioinformatics,* 2009, vol. 25, 4-10
- **STRICHMAN-ALMASHANU, L. Z. ; LEE, R. S. ; ONYANGO, P. O. ; PERLMAN, E. ; FLAM, F. ; FRIEMAN, M. B. ; FEINBERG, A. P.** A genome-wide screen for normally methylated human CpG islands that can identify novel imprinted genes. *Genome research,* 2002, vol. 12, 543-554